# EUROPEAN PATENT SPECIFICATION

(11) **EP 2 040 724 B1**
(45) Date of publication and mention of the grant of the patent: **05.10.2011**
(21) Application number: 07777134.3
(22) Date of filing: 17.05.2007
(51) Int. Cl.: A61K 31/4164, A61P 31/04, A61P 1/00

(54) **BIOTHERAPEUTIC COMPOSITIONS COMPRISING PROBIOTIC ESCHERICHIA COLI AND METRONIDAZOLE AND USES THEREOF**
BIOTHERAPEUTISCHE ZUSAMMENSETZUNG MIT PROBIOTISCHEN ESCHERICHIA COLI UND METRONIDAZOL UND IHRE VERWENDUNGEN
COMPOSITIONS BIOTHÉRAPEUTIQUES COMPRENANT DU ESCHERICHIA COLI PROBIOTIQUE ET DE LA MÉTRONIDAZOLE, ET LEURS UTILISATIONS

(30) Priority: 18.05.2006 US 801096 P
(43) Date of publication of application: 01.04.2009
(73) Proprietor: BIOBALANCE LLC, Brooklyn NY 11223 (US)
(72) Inventor: FITZPATRICK, Leo, York, PA 17402 (US); HOERR, Robert, A., Shoreview, Minnesota 55126 (US); BOSTWICK, Eileen, F., Dayton, Minnesota 55327 (US)
(74) Representative: Wachenfeld, Joachim
(86) International application number: PCT/US2007/011859
(87) International publication number: WO 2007/136719

(56) References cited:
- WO-A-02/43649
- FITZPATRICK LEO R ET AL: "Effects of the probiotic E-coli strain M-17 on chronic dextran sulfate sodium-induced colitis in mice" GASTROENTEROLOGY, ELSEVIER, PHILADELPHIA, PA, US, vol. 130, no. 4, Suppl 2, April 2006 (2006-04), page A313, XP009092064 ISSN: 0016-5085
- SCHROEDER B ET AL: "Preventive effects of the probiotic Escherichia coli strain Nissle 1917 on acute secretory diarrhea in a pig model of intestinal infection." DIGESTIVE DISEASES AND SCIENCES APR 2006, vol. 51, no. 4, April 2006 (2006-04), pages 724-731, XP002473763 ISSN: 0163-2116
- TROMM A ET AL: "THE PROBIOTIC E. COLI STRAIN NISSLE 1917 FOR THE TREATMENT OF COLLAGENOUS COLITIS: FIRST RESULTS OF AN OPEN-LABEL-TRIAL" ZEITSCHRIFT FUER GASTROENTEROLOGIE, XX, XX, vol. 42, no. 5, May 2004 (2004-05), pages 365-369, XP009063168 ISSN: 0044-2771
- BOZDOGAN BÜLENT ET AL: "Chromosomal aadD2 encodes an aminoglycoside nucleotidyltransferase in Bacillus clausii." ANTIMICROBIAL AGENTS AND CHEMOTHERAPY APR 2003, vol. 47, no. 4, April 2003 (2003-04), pages 1343-1346, XP002473764 ISSN: 0066-4804
- MATHUR SHALINI ET AL: "Antibiotic resistance in food lactic acid bacteria--a review." INTERNATIONAL JOURNAL OF FOOD MICROBIOLOGY 15 DEC 2005, vol. 105, no. 3, 15 December 2005 (2005-12-15), pages 281-295, XP005186597 ISSN: 0168-1605

## Description

### BACKGROUND OF THE INVENTION

The present invention relates to biotherapeutic compositions, and more particularly to compositions and uses thereof as referred in claims 1 - 9.

Anaerobic bacteria are bacteria which cannot grow in the presence of oxygen. Such bacteria can infect deep wounds, deep tissues, and internal organs where there is little oxygen, particularly when a normal barrier (such as skin, gums, or intestinal wall) is damaged due to surgery, injury, or disease. These infections are characterized by abscess formation, foul-smelling pus, and tissue destruction. Of the normal intestinal microflora, approximately 99.9 % are anaerobes. These include *Bacteroides, Prevotella, Clostridium, Peptostreptococcu, Escherichia, Proteus* and *Pseudomonas* as well as other less numerous species. The normal non-pathogenic flora competes with pathogens for nutrients and intestinal receptor sites, preventing them from causing disease.

The gastrointestinal tract represents a complex ecosystem in which a delicate balance exists between the intestinal microflora and the host. The microflora are principally comprised of facultative anaerobes and obligate anaerobes. Approximately 95 % of the intestinal bacterial population in humans is comprised of obligate anaerobes, including *Bifidobacterium, Clostridium, Eubacterium, Fusobacterium, Peptococcus, Peptostreptococcus* and *Bacteroides.* Approximately 1 % to 10 % of the intestinal population is comprised of facultative anaerobes, including *Lactobacillus, Escherichia coli, Klebsiella, Streptococcus, Staphylococcus* and *Bacillus.* Aerobic organisms are not present in the intestinal tract of healthy individuals with the exception of *Pseudomonas,* which is present in very small amounts. Most of the bacteria are present in the colon where the bacterial concentration ranges from 10¹¹ to 10¹² colony-forming units (CFU) per milliliter.

The intestinal microflora are important for maturation of the immune system, the development of normal intestinal morphology and in order to maintain a chronic and immunologically balanced inflammatory response. The microflora reinforce the barrier function of the intestinal mucosa, helping in the prevention of the attachment of pathogenic microorganisms and the entry of allergens. Some members of the microflora may contribute to the body's requirements for certain vitamins, including biotin, pantothenic acid and vitamin B₁₂. Alteration of the microbial flora of the intestine, such as may occur with antibiotic use, disease and aging, can negatively affect its beneficial role.

The interactions between the mucosal immune system and the enteric microflora maintain the physiologically normal state of inflammation or activation of gut-associated lymphoid tissue. In gastrointestinal disorders, colonic bacterial flora are implicated in the initiation and maintenance of intestinal dysfunctions, such as inflammatory bowel diseases and irritable bowel syndrome. Other intestinal disease states involving altered microflora include small-bowel bacterial overgrowth, colorectal cancer, and children's, traveler's and antibiotic-associated diarrhea.

Inflammatory bowel disease, or IBD, is a collective term encompassing related, but distinct, chronic inflammatory disorders of the gastrointestinal tract, such as Crohn's disease, ulcerative colitis (UC), indeterminate colitis, microscopic colitis and collagenous colitis, with Crohn's disease and ulcerative colitis being the most common diseases.

Ulcerative colitis is confined to the large intestine (colon) and rectum, and involves only the inner lining of the intestinal wall.

Crohn's disease may affect any section of the gastrointestinal tract (e.g., mouth, esophagus, stomach, small intestine, large intestine, rectum and anus) and may involve all layers of the intestinal wall. The disease begins with inflammation, most often in the lower part of the small intestine or in the colon, but sometimes in the rectum, stomach, esophagus or mouth. Unlike ulcerative colitis, in which inflammation occurs uniformly throughout an affected area, Crohn's disease can develop in several places simultaneously, with healthy tissue in between. In time, large ulcers that extend deep into the intestinal wall may develop in the inflamed areas. Complications such as obstruction of the bowel; ulcers anywhere in the digestive tract, including the mouth or the anus; fistulas; anal fissures; and malnutrition due to diarrhea, abdominal pain and cramping, may occur.

Both diseases, as well as other IBDs, are characterized by abdominal pain and cramping, diarrhea, rectal and/or intestinal bleeding, weight loss and fever. The symptoms of these diseases are usually progressive, and sufferers typically experience periods of remission followed by severe flare-ups. Less frequent, but also possible, IBD symptoms reflect mucosal inflammation of other sections of the GI tract, such as duodenitis, jejunitis and proctitis.

For most patients, IBD is a chronic condition with symptoms lasting for months to years. It is most common in young adults, but can occur at any age. It is found worldwide, but it is most common in industrialized countries such as the United States, England, and northern Europe. In fact, IBD affects an estimated two million people in the United States alone. Although IBD is not considered a fatal illness, prolonged disease can lead to severe malnutrition affecting growth or to the formation of abscesses or intestinal scar tissue, leading in turn to infection or bowel obstruction. Protracted IBD is also known as a risk factor for colon cancer.

Diagnosis of IBD is based on the clinical symptoms, the use of a barium enema (involving introduction of a contrast dye into the rectum in an enema form, which is visualized by x-rays), and/or direct visualization (sigmoidoscopy or colonoscopy), with the latter being the most accurate test, involving the visualization of the entire colon using a thin, flexible lighted tube with an attached camera. During colonoscopy, a biopsy sample may also be taken for laboratory analysis, mainly involving detection of granulomas, which are not present in ulcerative colitis. Sigmoidoscopy involves use of a slender, flexible, lighted tube to examine the sigmoid, the lower portion of the colon. Other diagnostic methods include small bowel X-ray, which views parts of the small bowel that cannot be seen by colonoscopy, by oral administration of barium in liquid form, followed by X-ray visualization; computerized tomography (CT), which views the entire bowel and surrounding tissue; and capsule endoscopy, which involves swallowing a capsule containing a camera, which transmits images to a computer, and are then downloaded, displayed on a monitor and checked for signs of Crohn's disease. For the diagnosis of Crohn's disease, see, for example, U.S. Patent Nos. 6,348,452 and 6,297,015.

The exact causes of IBD are not yet understood. Common hypotheses include, for example, disorders of the immune system, caused by a virus or bacterium, and actions of pro-inflammatory cytokines and selective activation of lymphocyte subsets, which perpetuate unrestrained activation of an inflammatory response in the intestine.

A hereditary factor is also believed to be involved, since it has been shown that about 20 percent of people with IBD disease have a parent, sibling or child who also has the disease. Mutations in the NOD2/CARD15 gene tend to occur frequently in people with Crohn's disease and seem to be associated with an early onset of symptoms as well as a high risk of relapse following surgery for the disease. It has also been suggested that since IBD occurs more frequently among people living in cities and industrial nations, it is possible that environmental factors, including a diet high in fat or refined foods, may play a role.

Presently, IBD has no cure. Patients afflicted with IBD are generally treated with therapies that are directed at reducing the inflammatory processes, and at reducing the effects of the inflammatory processes on the patients. The presently known medical treatment of IBD is intended to decrease the number, frequency and severity of acute exacerbations of inflammatory bowel disease and to prevent secondary complications, but at best, the results are disappointing. The presently known methods for treating IBD have involved anti-inflammatory drugs, immunosuppressive drugs, antibiotics and surgery.

The most commonly used medications to treat IBD are anti-inflammatory drugs such as the salicylates. Preparations of salicylate are effective in treating mild to moderate disease and can also decrease the frequency of disease flares when the medications are taken on a prolonged basis. Examples of salicylates include sulfasalazine, olsalazine, and mesalamine. Particularly, sulfasalazine and related drugs having the bioactive 5-amino-salicylic acid (5-ASA) moiety are widely used to control moderate IBD symptoms and to maintain remission. All of these medications are given orally in high doses for maximal therapeutic benefit. However, treatments with these medications is typically accompanied with adverse side effects such as nausea, dizziness, changes in blood chemistry (including anemia and leukopenia), skin rashes and drug dependence.

Corticosteroids are more potent and faster-acting anti-inflammatory drugs in the treatment of IBD, as compared with salicylates, and reduce inflammation anywhere in the body. Prednisone, for example, is a corticosteroid commonly used in the treatment of severe cases of IBD. Nevertheless, potentially serious side effects limit the use of corticosteroids to patients with more severe disease. Side effects of corticosteroids include puffiness of the face, excessive facial hair, night sweats, insomnia and hyperactivity. More serious side effects, which usually occur upon long term use, include high blood pressure, type 2 diabetes, osteoporosis, bone fractures, thinning of the bone and skin, muscle wasting, cataracts, increased susceptibility to infections, psychiatric disturbances, and, on rare occasions, destruction of hip joints. Long-term use of steroids in children can lead to stunted growth. A newer type of corticosteroid, budesonide, is metabolized faster than traditional steroids and appears to produce fewer side effects, but is effective only in Crohn's disease that involves the lower small intestine and the first part of the large intestine. Corticosteroids are unsuitable for long-term use. They can be used for short-term symptom improvement for about three to four months. They are also used in conjunction with other medications as a means to induce remission. For example, corticosteroids may be used with an immune system suppressor because the corticosteroids can induce remission, while the immune system suppressors can help maintain remission.

In cases where IBD patients do not respond to salicylates or corticosteroids, medications that suppress the immune system, namely immunosuppressants, are used. These drugs also reduce inflammation, but by targeting the immune system rather than treating the inflammation itself. Examples of immunosuppressants include azathioprine, 6-mercaptopurine, inflixmab, methotrexate, cyclosporine. However, as immunosuppressants may render the patient immuno-compromised and susceptible to other diseases, the use thereof in the treatment of IBD is not recommended. Furthermore, infliximab, which neutralizes tumor necrosis factor in the blood, is contraindicated for patients with heart failure, multiple sclerosis or cancer, and has also been linked to an increased risk of infection, especially tuberculosis, and may increase the risk of blood disorders and cancer. Infliximab can cause serious allergic reactions in some subjects. Short-term side effects of methotrexate include nausea, fatigue and diarrhea, and, occasionally, allergic pneumonia. Long-term use can lead to scarring of the liver and sometimes to cancer. Cyclosporine has the potential for serious side effects, such as kidney and liver damage, high blood pressure, fatal infections and an increased risk of lymphoma.

Antibiotics which are useful in the treatment of IBD include metronidazole, clarithromicin, tobramycin and ciprofloxacin.

Metronidazole is active against anaerobic bacteria and can be of benefit in the treatment of Crohn's disease of the ileum and colon, and in helping to prevent recurrences after surgical removal of part of the gut. Metronidazole is also used, off-label (namely, for indications not listed in the approved FDA labeling), in the treatment of various gastrointestinal infections, including, for example, *Clostridium* difficile-antibiotic-associated diarrhea infection, and small bowel overgrowth. To date, metronidazole has not been used in the treatment of inflammatory bowel diseases such as ulcerative colitis or Crohn's disease of organs other than the ileum and colon.

Metronidazole is distributed widely through body tissues both intracellularly and extracellularly. It is found in saliva and breast milk in concentrations equivalent to those in serum. It also crosses the placenta and is found in the CSF. Therapeutic levels have been found in abscesses, bile, CSF, seminal fluid and in synovial fluid. There is no significant plasma binding of metronidazole (less than 5 %). Metronidazole is partly metabolized in the liver by both acid oxidation and glucuronic conjugation. The half-life of metronidazole after single, intravenous infusion has been reported as 7.3 ± 1.0 hours.

Clarithromycin is active against *Helicobacter* which is associated with peptic ulcers. Tobramycin has limited use in severely ill patients with ulcerative colitis. Ciprofloxacin has utility alone or combined with metronidazole in active Crohn's disease.

A number of other antibiotics may be prescribed to deal with specific problems, such as the treatment of diarrhea caused by an imbalance in the bacterial population. However, antibiotics, especially if taken for a period of more than two weeks, may provoke a form of colitis. Very commonly, prolonged use of antibiotics is associated with diarrhea that may be a direct irritative effect of the drug itself (antibiotic-associated diarrhea).

Additional medications which may be prescribed to relieve the signs or symptoms of inflammatory bowel disease include, anti-diarrheals (for example, a fiber supplement such as psyllium powder or methylcellulose can help relieve signs and symptoms of mild to moderate diarrhea by adding bulk to the stool, and loperamide may be effective for more severe diarrhea); laxatives (in cases wherein swelling causes the intestine to narrow, leading to constipation); pain relievers (such as acetaminophen), iron supplements (to counter iron deficiency anemia which may develop due to chronic intestinal bleeding), and vitamin B-12 injections (in cases wherein normal absorption is reduced or prevented due to inflammation of the terminal ileum which normally absorbs this vitamin).

In more severe cases or when the drug therapy fails to relieve the symptoms of IBD, surgical procedures are used. Typical surgical procedures include colectomy, proctocolectomy and ileostomy (See, Cecil Textbook of Medicine, 19th Edition, Wyngaarden et al, ed., 1992). These surgical treatments are radical procedures that often profoundly alter the everyday life of the patient.

Some patients who have had the colon and rectum removed have a reservoir or pouch fashioned from a loop of the small intestine to serve in place of the rectum. Pouchitis is acute inflammation of the surgically produced pouch, which has been found to occur in between 7 and 50 percent of patients, depending on the quality and duration of follow-up.

The diagnosis of pouchitis has been defined using various criteria. Some researchers have favored a diagnosis based on clinical symptoms, while others recommended the use of endoscopic or histological features. Recently, it has been advocated that an unequivocal diagnosis should be based on a diagnostic trial consisting of clinical symptoms, endoscopic features of inflammation and histological evidence of inflammatory infiltrate. Endoscopically, the condition is characterized by oedema, granularity, mucous exudate, contact bleeding and ulceration and histological characteristics are chronic inflammatory infiltrate, crypt abscesses and ulceration.

Clinical symptoms of pouchitis include sudden onset of diarrhea, abdominal cramping and bloating, rectal urgency, tenesmus and incontinence, often with constitutional symptoms causing anorexia, generalized fatigue/malaise, fever, nausea and flu-like illness. Bowel frequency may be more than 30 times a day. Frequently there is blood, and occasionally pus, with the diarrhea. If the patient has had previous extra-intestinal manifestations of colitis these extra-intestinal manifestations often relapse with pouchitis, although they may sometimes present for the first time during an attack of pouchitis. In some cases, it can also result in joint pain and weight loss. Pouchitis has been reported to evoke arthritis, skin lesions and eye problems, resembling the extra-intestinal manifestations of inflammatory bowel disease (*Schouten, 1998).*

Endoscopic criteria for pouchitis are well known indicators of an acute nonspecific inflammation: granularity, oedema, erythema, friability, petechiae, hypersecretion and multiple superficial erosive defects. In the majority of cases, the endoscopic features of pouchitis mimic those of ulcerative colitis. Histological studies have revealed a chronic inflammatory filtrate in the lamina propria, including lymphocytes, plasma cells, eosinophils and histiocytes in the majority of patients.

Fecal statis with bacterial overgrowth has been considered a major contributing factor in the pathogenesis of pouchitis. Ileal reservoirs are colonized with large numbers of bacteria that outnumber the flora of the normal intestinal ileum. In ileal reservoirs without signs of pouchitis, the microflora closely resembles the flora of the large bowel, mainly due to the large numbers of anaerobes, resulting in a greater ratio of anaerobes to aerobes. It has been suggested that incomplete emptying of the pouch, which is associated with statis of ileal contents, would result in an increase in the number of anaerobic bacteria.

The cause of pouchitis is not known. Several theories have been suggested, including an excess of bacteria, such as sulfate-reducing bacteria, in the pouch; chronic ischemia; deficiency of short chain fatty acids; stasis; immunological reactivation; and a recurrence of inflammatory bowel disease in the pouch and misdiagnosis of ulcerative colitis. Possible factors that affect the likelihood of developing pouchitis include the presence of sclerosing cholangitis; use of non-steroidal anti-inflammatory drugs (NSAIDs); post-operative administration of prednisone or immunosuppressants; and smoking status of the patient. It is considered that bacterial enzymes, such as glycosidases, degrade the protecting mucus, which may become more permeable to toxic bacterial metabolites and host-derived proteolytic enzymes, affecting the integrity of the mucosa. As a result, bacterial antigens may cross the mucosal barrier. This translocation of bacterial antigens probably triggers a cascade of inflammatory events, which result in pouchitis.

Pro-inflammatory cytokines, which include interleukin-1β, TNF-α, IL-8 and IL-12, appear to play an important role in pouch inflammation. Signal transduction of cytokine/growth factor receptors involves the activation of transcription factors as part of the signal transduction pathway. The control of transcriptional events is exerted at specific sites in gene promoter regions. A significant increase in the expression and activation of the pro-inflammatory transcription factor STAT 1 (signal transducer and activator of transcription) has been reported in biopsies from pouchitis patients in comparison with both uninflamed pouch mucosa and normal preoperative ileum. STAT proteins are dormant cytoplasmic transcription factors which are phosphorylated in response to activation of cytokine and growth factor receptors. Activation may involve IFN-γ (*Kuhbacher, 2001*). Activated STAT1 proteins translocate into the nucleus and can augment transcription by binding to specific DNA sequences in gene promoter regions of specific genes.

Pouchitis occurs more commonly in people with extraintestinal problems associated with ulcerative colitis (e.g. arthritis or abnormalities of the liver, skin, or eyes). These findings suggest that pouchitis may be a new type of IBD, which occurs in the pouch. The majority of individuals with pouchitis do not have Crohn's disease.

Individuals with pouchitis often improve with antibiotics, particularly metronidazole or ciproflaxin, suggesting that bacteria are an important factor in the development of this condition. The study of pouch bacterial flora has shown an increase in the total number of bacteria and in the ratio between anaerobic and aerobic bacteria (*Nasmyth, 1989*). The beneficial effect of antibiotics in pouchitis suggests that an unidentified fecal bacterial product causes this condition. A candidate compound is hydrogen sulfide, a highly toxic gas produced by certain fecal bacteria, which causes tissue injury in experimental models.

Alternative treatments for pouchitis include topical mesalamine (as a suppository or enema); oral and topical corticosteroids; and immunosuppressive medications, such as infliximab.

Probiotics are a class of microorganisms defined as live microbial organisms that beneficially affect the animal and human hosts. The beneficial effects include improvement of the microbial balance of the intestinal microflora or improving the properties of the indigenous microflora. The beneficial effects of probiotics may be mediated by a direct antagonistic effect against specific groups of organisms, resulting in a decrease in numbers, by an effect on their metabolism or by stimulation of immunity. The mechanisms underlying the proposed actions remain vastly unknown, partly as a consequence of the complexity of the gastro-intestinal ecosystem with which these biotherapeutic agents are expected to interact. Probiotics may suppress viable counts of an undesired organism by producing antibacterial compounds, by competing for nutrients or for adhesion sites. Further, they may alter microbial metabolism by increasing or decreasing enzyme activity or they may stimulate the immune system by increasing antibody levels or increasing macrophage activity. Probiotics may have antimicrobial, immunomodulatory, anti-carcinogenic, anti-diarrheal, anti-allergenic and antioxidant activities. Known probiotic strains include, for example, *Bifidobacteria, Lactobacillus, Lactococcus, Saccharomyces, Streptococcus thermophilus, Enterococcus* and *E. coli.*

It is well known in the art that under conditions where the balance of the GI microflora is adversely affected, probiotics become of potential value in restoring the GI microflora and enabling the individual host to return to normal. Treatments of various GI disorders using probiotic compositions are disclosed, for example, in WO 95/16461 and in WO 97/35596.

Bacterial endotoxins have been detected in the plasma of IBD patients, and an abnormal microflora and/or an increased permeability of the intestinal mucosa has been speculated to be responsible for endotoxemia (*Caradonna, 2000*). Probiotics may induce a "barrier" influence against common pathogens and the mechanisms are likely to include the excretion of acids (lactate, acetate), competition for nutrients and gut receptor sites, immunomodulation, and the formation of specific antimicrobial agents.

Oral probiotic treatment has been suggested for ulcerative colitis. The effects have been attributed to the oxidation of short-chain fatty acids in colonocytes and to the ability of butyrate to induce enzymes (i.e. transglutaminase) promoting mucosal restitution. The relative efficacy of different probiotics could be based on their characteristics, which include survival, adhesion and colonization. For example, the probiotic *Escherichia coli* Nissle 1917 has been shown to be effective as mesalazine in maintaining remission of ulcerative colitis (*Kruis*, 2004).

Immunomodulatory actions, such as the reduction of pro-inflammatory cytokines (e.g., TNF-α, IFN-γ), and the increased secretion of regulatory cytokines (e.g., IL-10), have been suggested as one of the mechanisms of action for probiotics (*Bauerle, 1994*). The transcription factor NF-κB, once separated from its inhibitory protein (IκB), translocates into the nucleus where it activates genes encoding immunologically relevant proteins (e.g., TNF-α, IL-1β, and IL-6; *Bauerle*, *1994; Baldwin*, 1996; *Tak, 2001*). NF-κB is thought to play a key role in the pathogenesis of intestinal inflammation. Evidence supporting the pro-inflammatory role of NF-κB comes from both animal models of enteric inflammation and from patients with IBD. It is not surprising, therefore, that the inhibition of NF-κB has been proposed as an important therapeutic target for IBD (*Jobin, 2000a; Jobin, 2000b; Lee, 1998_{:} Schottelius, 1999*). Recently, it was reported that probiotics could inhibit the NF-κB signal transduction system (*Araki*, *2004; Osman*, *2004*).

Several different probiotic agents have demonstrated evidence of efficacy in the dextran sulfate sodium (DSS)-induced colitis model (*Araki, 2004; Osman, 2004*). The pathogenesis of DSS-induced colitis involves a defect in epithelial barrier function, as related to the direct cytotoxic effect of DSS (*Egger*, *2000*; *Strober, 2002*). Changes in epithelial barrier function, as measured by the permeability to Evan's blue dye, can be found early during the time course of DSS-induced colitis (*Kitajima. 1999).* This alteration in the colonic mucosal barrier subsequently leads to the influx of various inflammatory ceils, macrophage activation and pro-inflammatory cytokine production (*Egger*, *2000; Strober, 2002*). Additionally, the nuclear expression of the p65 subunit of NF-κB has previously been shown to be up-regulated during DSS-induced colitis and is thought to play a critical role in promoting intestinal inflammation (*Murano, 2000; Spiik, 2002*).

U.S. Patent Application having the Publication No. 20020006432, to Collins et al., teaches a strain of Bifidobacterium isolated from resected and washed human gastrointestinal tract which is said to be significantly immunomodulatory following oral consumption in humans. The strain is taught to be useful in the prophylaxis and/or treatment of undesirable inflammatory activity, especially gastrointestinal inflammatory activity such as inflammatory bowel disease or irritable bowel syndrome.

Recently, it was uncovered that a single species of a non-pathogenic probiotic microorganism derived from *E. coli* is, alone, capable of restoring normal GI flora of human and of a variety of mammals and avians. The beneficial physiological and therapeutic activity of this species in the GI tract is described in detail in U.S. Patent No. 6,500,423, and in WO 02/43649. These references teach that the *Escherichia coli* strain BU-230-98 ATCC Deposit No. 202226 (DSM 12799), which is an isolate of the commercially available probiotic *E. coli* M17 strain, is highly effective in preventing or treating gastro-enteric infections or disorders, maintaining or reinstating normal gastrointestinal microflora, preventing or treating diarrhea, preventing or treating gastro-enteric infection caused by an enteric pathogen, such as a Gram negative bacterium or Gram positive bacterium, preventing or treating gastro-enteric *Salmonella* infection, preventing or treating infectious diarrhea, caused by, for example *C*. *difficile*, *Salmonella,* particularly *Shigella, Campylobacter, E. coli, Proteus, Pseudomonas* or *Clostridium* or diarrhea resulting from antibiotic therapy, radiotherapy or chemotherapy, and/or for formalizing the physiological activity of the gastrointestinal tract. Furthermore, U.S. Patent No. 7,018,629 teaches that strain BU-230-98 ATCC Deposit No. 202226 (DSM 1.2799), while altering the microbial balance in the GI tract, is highly efficacious agent for treating IBD, such as Crohn's diseases and the symptoms associated therewith and for treating other idiopathic inflammation of the small and proximal intestine.

Fitzpatrick *et al.* 2006 (Gastroenterology 130:A313) describes the efficacy profile of the probiotic *E. coli* strain M-17 on chronic DSS-induced colitis in mice and concludes that this probiotic has a similar efficacy profile as metronidazole.

However, in some cases, the use of a probiotic microorganism alone is not sufficient to treat all cases of gastro-enteric inflections or disorders. There is thus a widely recognized need for and it would be highly advantageous to have improved compositions for use in the treatment of intestinal disorders such as IBD and pouchitis.

### SUMMARY Of THE INVENTION

The prior art does not teach or suggest a composition comprising a non-pathogenic bacterial strain and an antibiotic. The background art also does not teach or suggest such a composition for treatment of various intestinal disorders, including but not limited to, microbial infection, irritable bowel syndrome (IBS), inflammatory bowel disease (IBD) and pouchitis.

According to one aspect of the present invention there is provided a biotherapeutic composition comprising a pharmaceutically effective amount of a probiotic *Escherichia coli* strain, a pharmaceutically effective amount of at least one anaerobic bacteria antibiotic to which said *Escherichia coli* strain is resistant, and a pharmaceutically acceptable carrier as characterised by the appended claims.

Further features described below include a concentration of *Escherichia coli* strain in the composition ranges from about 5 x 10⁷ to about 5 x 10⁹ colony forming units per 1 ml of carrier.

The carrier may comprise 0.6 % saline solution. Alternatively, the carrier may comprise other hypotonic solutions, which includes one or more highly water soluble salts are used (instead of or in addition to NACl).

The composition described may further comprise at least one flavoring agent.

The claimed composition is identified for use in the treatment or prevention of a condition caused by an anaerobic bacterium as characterised by the appended claims. Preferably, the composition is packaged in a packaging material and identified in print, in or on the packaging material, for use in the treatment or prevention of a condition caused by an anaerobic bacterium as characterised by the appended claims.

According to still another aspect of the present invention there is provided a use of a pharmaceutically effective amount of a probiotic *Escherichia coli* strain, in combination with a pharmaceutically effective amount of at least one anaerobic bacteria antibiotic to which the *Escherichia coli* strain is resistant, in the manufacture of a medicament for treating a condition caused by an anaerobic bacterium as characterised by the appended claims.

The antibiotic may be administered prior to, concomitant with or subsequent to administering the probiotic strain.

According to further features in the described preferred embodiments, the probiotic strain and the antibiotic are co-formulated in a biotherapeutic composition, as described herein.

According to further features in preferred embodiments of the invention described below, the *Escherichia coli* strain utilized in the composition or use is at least one of M-17 and any isolate or mutant thereof, such as, for example *Escherichia coli* strain BU-230-98 ATCC Deposit No. 202226 (DSM 12799); and a nalidixic acid resistant mutant derivative thereof such as *Escherichia coli* strain ATCC Deposit No: PTA-7295 (M17_{SNAR}).

According to further features in the described preferred embodiments, the anaerobic bacterium is a sulfate-reducing bacterium.

According to the features of the appended claims, the antibiotic is metronidazole. Preferably, the *Escherichia coli* strain is M-17 and the antibiotic is metronidazole.

According to the present embodiments, the *Escherichia coli* strain and the antibiotic act in synergy.

The composition or the use described herein optionally further comprises an additional active ingredient, such as an anti-inflammatory agent, an anti-diarrheal agent, a laxative, a pain reliever, an iron supplement, a probiotic, and an immunosuppressive agent, co-formulated in the composition or utilized along with the E. coli strain and the antibiotic.

According to the present embodiments, the condition treatable by the compositions or uses of the present invention is an intestinal disorder, such as pouchitis, microbial infection, irritable bowel syndrome, inflammatory bowel disease, mucous colitis and diarrhea. Preferably, the condition is pouchitis.

According to still further features in the described preferred embodiments, a pharmaceutically effective amount of metronidazole preferably ranges from about 5 to about 50 mg per kg body weight of said subject, per day.

A pharmaceutically effective amount of olsalazine, (not part of the invention) preferably ranges from about 10 to about 50 mg per kg body weight of said subject, more preferably about 15 mg per kg body weight, per dry.

The present invention successfully addresses the shortcomings of the presently known configurations by providing a composition for treating and preventing discorders caused by anaerobic bacteria, which combine a probiotic *E. coli* strain and an anaerobic bacteria antibiotic as referred to in claims 1-9. Such treatment is highly advantageous as compared with the present methods of treating such disorders, as it is efficacious, safe, non-invasive and side effect-free.

Unless otherwise defined, all technical and Scientific terms used herein have the some meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

The term "comprising" means that other steps and ingredients that do not affect the final result can be added. This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

The term "therapeutically effective amount" or "pharmaceutically effective amount" denotes that dose of an active ingredient or a composition comprising the active ingredient that will provide the therapeutic effect for which the active ingredient is indicated.

As used herein a "pharmaceutical composition" refers to a preparation of one or more of the active ingredients described herein, either compounds or physiologically acceptable salts thereof, with other chemical components such as traditional drugs, physiologically suitable carriers and excipients.

As used herein, the term "pharmaceutically acceptable" means approved by a regulatory agency of the Federal or a state government or listed in the U.S. Pharmacopeia or other generally recognized pharmacopeia for use in animals, and more particularly in humans. Herein, the phrases "physiologically suitable carrier" and "pharmaceutically acceptable carrier" are interchangeably used and refer to an approved carrier or a diluent that does not cause significant irritation to an organism and does not abrogate the biological activity and properties of the administered conjugate.

As used herein, the singular form "a," "an," and "the" include plural references unless the context clearly dictates otherwise. For example, the term "a compound" or "at least one compound" may include a plurality of compounds, including mixtures thereof.

Throughout this disclosure, various aspects of this invention can be presented in a range format.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a bar graph presenting the results of M-17 and metronidazole treatment on colonic IL-12 levels in saline/DSS-treated mice, as compared to saline/water treated mice as a control;
FIG. 2 is a bar graph presenting the results of M-17 and metronidazole treatment on colonic IFN-γ levels in saline/DSS-treated mice, as compared to saline/water treated mice as a control;
FIG. 3 is a bar graph presenting the results of M-17 and metronidazole treatment on colonic IL-1β levels in saline/DSS-treated mice, as compared to saline/water treated mice as a control;
FIG. 4 is a bar graph presenting the results of M-17 and metronidazole treatment on colonic IL-6 levels in saline/DSS-treated mice, as compared to saline/water treated mice as a control;
FIG. 5 is a bar graph presenting the results of M-17 and metronidazole treatment on colonic IL-10 levels in saline/DSS-treated mice, as compared to saline/water treated mice as a control;
FIG. 6 is a bar graph presenting the results of M-17 and metronidazole treatment on colonic IL-4 levels in saline/DSS-treated mice, as compared to saline/water treated mice as a control;
FIG. 7 is a bar graph presenting the results of intestinal permeability studies in saline/DSS-treated mice, as compared to saline/water treated mice as a control;
FIG. 8 is a bar graph presenting Disease Activity Indices for DSS-treated mice and control mice, treated and untreated with M-17;
FIG. 9 presents a plot showing the correlation between Disease Activity Indices and intestinal permeability in control (saline treated) animals;
FIG. 10 is a bar graph presenting normalized colonic permeability values in DSS-treated and control mice, treated and untreated with M-17;
FIG. 11 is bar graph presenting the results of M-17 and metronidazole treatment on total colonic histology studies in saline/DSS-treated mice, as compared to saline/water treated mice as a control;
FIGs. 12A-12D show photographic results of colonic histology studies of mice treated with saline/water (Figure 12A); saline/DSS (Figure 12B); M-17 (Figure 12C); and M-17 plus metronidazole (Figure 12D);
FIG. 13 presents a Western Blot analysis demonstrating the effect of M-17 on up-regulation of LMP2 in DSS-treated mice;
FIG. 14 is a bar graph presenting colonic Western Blot demonstrating the effect of M-17 on up-regulation of LMP2 in DSS-treated mice;
FIGs. 15A-B present Western Blot analyses demonstrating the effect of M-17 on murine colonic p65 expression in mice given water for 13 days (Figure 15A) or DSS for 13 days (Figure 15B);
FIGs. 16A-B are bar graphs presenting the results of a study of the effects of M-17 in a NF-κB Reporter Gene Assay at various concentrations of M-17 (Figure 16A) and with a negative (non-TNF) control (Figure 16B);
FIG. 17 presents a schematic representation of cytokine secretion by activated macrophages;
FIG. 18 is a bar graph presenting IL-1β levels in macrophages exposed to LPS or saline, in the presence and absence of various *E. coli* strains;
FIG. 19 is a bar graph presenting TNF-α levels in macrophages exposed to LPS or saline, in the presence and absence of various *E*. *coli* strains;
FIG. 20 is a bar graph presenting IL-6 levels in macrophages exposed to LPS or saline, in the presence and absence of M17;
FIGs. 21A-21C are bar graphs presenting the effects of M-17, M-17 conditioned medium (CM) and heat-killed M-17 on the secretion of the cytokines TNF-α (Figure 21A), IL-1β (Figure 21B) and IL-6 (Figure 21C); and
FIG. 22 is a bar graph presenting the effect of M-17 on NF-κB p65 in macrophages exposed to LPS or saline vehicle, using a wild-type oligonucleotide which blocks p65 binding as a control.

### DETAILED DESCRIPTION OF THE INVENTION

The present invention is of a novel biotherapeutic composition containing a probiotic bacterial strain and an antibiotic to which the bacterial strain is resistant, which can be efficiently used in treating or preventing a condition caused by anaerobic bacteria as referred to in claims 1-9. Specifically, the present invention is of a novel biotherapeutic composition, which comprises a probiotic *Escherichia coli* strain and an anaerobic bacteria antibiotic such as metronidazole, and of uses thereof in the treatment of gastrointestinal disorders such as pouchitis as characterised by the appended claims.

The principles and operation of the biotherapeutic composition and the method according to the present invention may be better understood with reference to the drawings and accompanying descriptions.

Before explaining at least one embodiment of the invention in detail, it is to be understood that the invention is not limited in its application to the details set forth in the following description or exemplified by the Examples.

As discussed hereinabove, in the normal, healthy gastrointestinal tract a delicate balance exists between the intestinal microflora, which largely comprises anaerobic organisms (both facultative and obligate) and the host.

It has been found that under conditions where the balance of the microflora of gastrointestinal tract is adversely affected, restoration of the normal microbalance, by the introduction of probiotic organisms normally found in the GI tract has a beneficial effect.

As discussed hereinabove, *E. coli* bacteria are normal colonists of the human gastrointestinal tract. Non-pathogenic probiotic microorganisms derived from *E. coli* have been found to be particularly useful in restoring normal gastrointestinal flora of a variety of various mammals, including humans.

Various antibiotics which are effective against anaerobic bacteria have been used to treat anaerobic infections of the gastrointestinal tract. These include, for example, metronidazole, clarithromicin, tobramycin and ciprofloxacin. However, it has been found that many anaerobic bacteria antibiotics, such as ciprofloxacin, in addition to their beneficial effect in destroying undesirable bacteria, also act against probiotic bacteria, including those which are naturally present in the gastrointestinal tract, and those which are administered in order to restore a microbial imbalance. The beneficial probiotic effect of an administered *E*. *coli* strain would therefore be lost upon joint administration of such strains with an anaerobic antibiotic, resulting in little or no added advantage over the use of either ingredient alone.

The present inventors have postulated that by selecting a particular probiotic strain and an anaerobic bacteria antibiotic to which the strain is resistant, a highly effective novel biotherapeutic composition may be provided. The use of such a combination is neither taught nor suggested by the prior art.

It was further postulated that a biotherapeutic composition comprising a probiotic *Escherichia coli* strain and an anaerobic bacteria antibiotic to which the *Escherichia coli* strain is resistant would be particularly effective in the treatment of various conditions caused by anaerobic bacteria, especially those of the gastrointestinal tract, such as, for example, pouchitis.

The development of resistance of microorganisms to antibiotics may involve modification or elimination of the interaction between the antibiotic and target molecule, or alteration or regulation of the target molecule to prevent or overcome this interaction. Bacteria acquire genes conferring resistance in any of three ways: by spontaneous DNA mutation, resulting in reduced transcription of molecules that mediate the antibiotic-target interaction, thereby decreasing effectiveness of the antibiotic within the cell; by transformation, in which one bacterium takes up DNA from another bacterium; and by plasmid transfer. The bacterial microcosm present in the gastrointestinal tract provides an excellent opportunity for the transfer of antibiotic resistance genes, with the normal intestinal flora acting as a reservoir for such resistance traits. Native *E. coli,* unexposed to antibiotic pressures, tend to be fully sensitive to all antibiotics and other antimicrobial agents, but exposure to such substances will cause the development of resistance. Both plasmid mediated and chromosomally determined resistances have been described. A chromosomal regulatory locus in *Escherichia coli, Shigella, Salmonella* and other members of the *Enterobacteriaceae* simultaneously controls intrinsic levels of susceptibility to structurally different antibiotics (tetracyclines, chloramphenicols, penicillins, nalidixic acid, fluoroquinolones, etc.) and disinfectants (triclosan, quaternary ammonium compounds).

The present inventors have surprisingly found that the combination of certain *E. coli* strains and anaerobic bacteria antibiotics results in a synergistic effect, such that a significantly greater efficacy was produced as compared to treatment by either component alone.

According to one aspect of the present invention, there is provided a biotherapeutic composition comprising a phamarceutically effective amount of a probiotic *Escherichia coli* strain, a pharmaceutically effective amount of at least one anaerobic bacteria antibiotic to which said *Escherichia coli* strain is resistant, and a pharmaceutically acceptable carrier as characterised by the appended claims.

As used herein, the term "probiotics" describes isolated bacteria having the property of inhibiting the growth of at least one pathogen. Inhibition may be tested by any method known to the art, such as an in vitro test on solid medium in which culture supernatants or candidate isolated bacteria are observed for their property of inhibiting the growth of a pathogen when applied to the surface of the send medium. Typically, a paper disc impregnated with the culture supernatant of a candidate probiotic strain is placed on the surface of an agar plate seeded with the pathogen. Probiotic bacterial supernatants cause a ring of clear agar or of reduced growth density, indicating inhibition of the pathogen in the vicinity of the disc. Other tests for inhibition are available or could be devised, including direct growth competition tests, in vitro or in vivo, which can generate, a panel of probiotic bacteria similar to that described herein.

As discussed hereinabove, the *Escherichia coli* strain identified by ATCC Deposit No. 202226 (DSM 12799), which is an isolate of the commercially available probiotic *E*. *coli* M-17 strain, has been previously found to be highly effective in preventing or creating a wide variety of gastro-enteric injections or disorder, and in mainlining or reinstating normal gastro-intestinal microflora, and/or in normalizing the physiological activity of the gastrointestinal tract. Conditions treatable by this strain include, for example, inflammatory bowel disease, such as Crohn's disease, and the symptoms associated therewith, and idiopathic inflammation of the small and proximal intestine. It was therefore considered that preferred probiotic *E*. *coli* strains for use according to the teachings of present invention include non-pathogenic *E*. *coli* strains which exert probiotic activity. The presently most preferred probiotic *E. coli* strain is the strain M-17, including sub-strains thereof. Examples of M-17 strains include but are not limited to, BU-239, BU-230-98, BU-230-01, BU-230-98 ATCC Deposit No. 202226 (DSM 12799) and/or a nalidixio acid resistant mutant derivative of the E. coli M-17 strain.

Such an M-17 nalidixio acid resistant strain (e.g., the strain deposited under ATCC Deposit No. PTA-7295), also referred to herein as M17_{SNAR}, is a mutant derivative strain obtained by growing strain BU-230-98 on sucrose and nalidixic acid. Unlike many other *E*. *coli* strains, M-17 strains are sucrose resistant, and the strain obtained is also resistant to nalidixic acid and is thus referred to as M17_{SNAR} (Sucrose Nalidixic Acid Resistant). DNA from this strain has been isolated and the genome has been sequenced at the University of Minnesota (Biomedical Genomics Center). Additional information regarding the various features and activity of the M17_{SNAR} strain are disclosed in U.S. Provisionnal Patent Application No. 60/801,098.

As used herein, the term "anaerobic bacterium." refers to a bacterium which does not require the presence of oxygen for growth. These include both obligate anaerobes, i.e. those which can survive only in the absence of oxygen, and facultative anaerobes, i.e. those which can survive in the presence or absence of oxygen.

Preferably, the bacterium against which the antibiotic of the present invention is effective is a sulfate-reducing bacterium. Sulfate-reducing bacteria are anaerobic prokaryotes, which are members of the normal intestinal microbiota and have a major impact on terminal fermentative processes that occur in the mammalian colon. The metabolic pathways used by these bacteria result in the conversion of sulfate ions, SO₄⁻², into the highly reactive and toxic end-product sulfide, S⁻², with the concomitant oxidation of a carbon source, in the form of small organic molecules (e.g., lactate, pyruvate, acetate, and in a few cases, also alkanes and aromatic compounds). Intestinal sulfate can be derived either from exogenous sources, namely sulfate in drinking water and dietary foodstuffs, or from endogenous sources such as sulfated mucins (sulfomucins), sulfate-conjugated bile, and chondroitin sulfate.

Hydrogen sulfide selectively impairs the oxidation of *n*-butyrate by colonic epithelial cells. Because membrane lipid biosynthesis, ion absorption, mucin synthesis, and detoxification processes in colonocytes depend on the oxidation of *n-*butyrate, diminished *n*-butyrate metabolism is likely to compromise the epithelial cell barrier. Sulfide-induced damage of the epithelial barrier function would promote translocation of bacterial and food antigens, resulting in local inflammatory responses to normally benign antigens, an outcome consistent with histopathological features of IBD. Chronic exposure to H₂S might also perturb normal cycles of epithelial renewal in the intestine, thereby predisposing to proliferative disorders such as colon cancer.

The antibiotic of the present invention is an anaerobic bacteria antibiotic to which the *Escherichia coli* strain of the composition is resistant, metronidazole, (1-(beta-hydroxyethyl)-2-methyl-5-nitroimidazole), which has the following structural formula:

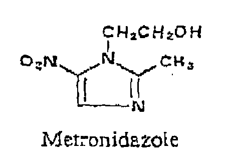

Metronidazole acts by inhibition of DNA synthesis. Metronidazole is amebicidal, trichomonacidal and bactericidal. The antibiotic is administered in an inactive form, entering the cells by diffusion. Metronidazole is reduced at the nitro group by intestinal bacteria, particularly anaerobes. A reactive intermediate is thereby formed which binds to critical sites in susceptible bacterial cells, with subsequent disruption of DNA and inhibition of its synthesis.

Metronidozole-resistance has been attributed to reduced transcription of one of the proteins involved in hydrogenosome localized reductive activation. The reduced substrate affects anoxic or hypoxic cells causing loss of the helical structure of DNA, strand breakage and impairment of cellular function.

The spectrum of activity of metronidazole includes anaerobic gram-negative *bacilli,* including most. *Bacteroides* species, *Fusobacterium* and *Veilionella*; anaerobic gram-positive cocci including, *Clostridium, Eubacterium, Peptococcus* and *Peptostreptococcus.* Metronidazole is also active against *H*. *pylori. G*. *vaginalis* and the protozoa *E. histolytica T. vaginalis* and *G*. *lamblia.* Metronidazole acts primarily against the trophozoite forms of *E*. *histolytica* and has limited activity against the encysted forms. Metronidazole is not active against fungi or viruses.

Metronidazole is currently indicated for the treatment of a variety of anaerobic infections such as abdominal infections, skin and tissue infections, bone and joint infections, gynecologic infections, and respiratory tract infections, primarily by inhibition of DNA, repair enzymes that normally repair cells of anaeorobic bacteria. Metronidazole can help heal fistulae associated with Crohn's disease by killing the bacteria present in the fecal matter contained in the fistulae. Metronidazole is also used in radiotherapy for cancer as this DNA offset can sensitize anaerobic tumor tissues to radiation masking a smaller dose of radiation more effective. Metronidazole also has anti-inflamatory properties in the large intestine, and is a very effective anti-diarrhea medication.

Use of a selected strain, together with an antibiotic to which that strain is resistant, enables each of the components to exert a beneficial effect against the condition being treated. Described herein is that this may be an additive effect, such that the effect of the probiotics is exerted in addition to the effect of the antibiotic. In accordance with the present embodiments, the *Escherichia coli* strain and the antibiotic of the composition produce a synergistic effect.

As used herein "synergy" or "synergistic effect" will regard to an effect produced by two or more individual components refers to a phenomenon in which the total effect produced by these components, when utilized in combination, is greater than the sum of the individual effects of each component acting alone

As shown in Example 3, Table 7, of the Examples section that follows, the effects of combined treatment with the antibiotic metronidazole, and the probiotic M-17 strain, on measured parameters of the acute phase DSS-colitis model, such as colonic levels of IL-12, IL-6, IL-1β, and IFN-γ, as well as changes in DAI, colon length, colon weight, MPO activity, and colonic histology score, were greater than that observed with either treatment alone, indicating a synergistic effect of the combined treatment.

Resistance of a probiotic strain to an antibiotic may be determined by any method known in the art, such as, for example, the Kirby-Bauer disc diffusion method described in the Examples section below with regard to Example 6.

As used a a "biotherapeutic composition" refers to a preparation comprising a microorganism having therapeutic properties. Such microorganisms should preferably be innocuous, act against pathogens by multiple mechanisms (thus minimizing the development of resistance), and marshal the host defenses to destroy invading pathogens. An additional desirable property would be an immediate onset of action. Microorganisms suitable for use in biotherapeutic compositions include yeast and bacterial isolates.

The term "pharmaceutically effective amount" refers to that amount of a probiotic *E. coli* strain or antibiotic which will relieve or prevent, to at least some extent, one or more of the symptoms of the condition being treated or prevented.

Metronidazole has been reported to be effective in treating pouchitis in humans at doses of about 1.2 gram per day (*Madden et al*., *1994*) or about 20 mg/kg per day (*Shen et al*., *2001*). These are approximately half the equivalent concentrations expressed in mg per kg body weight which were found to be effective in mice.

A pharmaceutically effective amount of the antibiotic used in the context of the present embodiments, wherein the antibiotic is metronidazole, therefore preferably ranges from about 5 mg per kg body weight of the subject to about 50 mg per kg body weight of the subject as a daily amount, more preferably from about 10 mg per kg body weight of the subject to about 30 mg per kg body weight of the subject daily, and most preferably is about 20 mg per kg body weight of the subject. Hence, for example, an adult human weighing approximately 70 kg, would preferably receive about 1.4 grams of antibiotic daily. This may be administered as a single daily dose, or in a number of divided doses, such as, for example, about 700 milligrams twice daily; about 450 milligrams thrice daily; or about 350 milligrams four times daily.

Metronidazole may be administered either orally or intravenously. Thus, for example, the antibiotic may optionally be administered by injection or infusion, such as by intravenous drip, preferably at a volume of about 5 ml/kg. A 70 kg individual would thus preferably receive a total of about 350 ml metronidazole solution per day. Hence, for example, metronidazole for infusion may optionally be provided in ready to use form in a 100 ml single dose plastic container, comprising a sterile, nonpyrogenic, iso-osmotic, buffered solution of 700 mg metronidazole in water, for injection. Optional excipients include, for example, sodium chloride, sodium phosphate; and citric acid. Metronidazole may optionally be supplied in lyophilized form, as single-dose vials containing sterile, nonpyrogenic Metronidazole HCl, equivalent to 750, 450 or 350 mg metronidazole, and mannitol. For infusion, metronidazole is preferably administered at a rate of about 5ml per minute for an infusion period of about 20 minutes.

Alternatively, metronidazole may be administered orally in the form of tablets containing, for example, 750 mg of metronidazole. Inactive ingredients include, for example, cellulose, FD&C Blue No. 2 Lake, hydroxypropyl cellulose, hydroxypropyl methylcellulose, polyethylene glycol, stearic acid, and titanium dioxide.

The compositions and uses described herein may further comprise an anti-inflammatory agent, such as olsalazine. As discussed in the Background section hereinabove, anti-inflammatory agents, such as olsalazine, are commonly used for treatment of IBDs, such as ulcerative colitis. Hence, use of a composition further comprising such an anti-inflammatory agent would be useful in the treatment of ulcerative colitis, while further preventing the development of pouchitis, which occurs as a complication of ulcerative colitis.

For compositions and uses comprising olsalazine as the anti-inflammatory agent, a pharmaceutically effective amount preferably ranges from about 10 mg per kg body weight of the subject to about 50 mg per kg body weight of the subject as a daily amount, more preferably from about 10 mg per kg body weight of the subject to about 35 mg per kg body weight of the subject daily, and most preferably is about 15 mg per kg body weight of the subject. Hence, for example, an adult human weighing approximately 70 kg, would preferably receive about 1 gram of olsalazine daily. This may be administered as a single daily dose, or in a number of divided doses, such as for example, about 250 milligrams four times daily, 6 times daily, 8 times daily, 10 times daily, or 12 times daily. Reference to common dosages and unit doses of olsalazine can be found, for example, in *Wolf and Lashner* (2002).

Olsalazine may be administered orally, for example in the form of a tablet or capsule. Hence, for example, olsalazine may optionally be administered in the form of hard gelatin capsules, comprising, for example, 250 mg per capsule of olsalazine sodium. The capsule may further comprise optional excipients, such as, for example, magnesium stearate.

The viable colony-forming unit count of the probiotic strain of the present embodiments should be sufficient to exert a biotherapeutic effect upon reaching the gastrointestinal tract. The carrier utilized in the composition according to the present embodiments should thus preferably be selected so as to maintain the viability of the microorganism for a prolonged period of time, at room temperature and/or when refrigerated, and to enable delivery of viable organisms to the gastrointestinal tract.

Herein, the term "pharmaceutically acceptable carrier" refers to a carrier or a diluent that does not cause significant irritation to a subject and does not abrogate the biological activity and properties of the administered active ingredient.

Herein the term "excipient" refers to an inert substance added to a pharmaceutical composition to further facilitate administration of the active ingredient.

The biotherapeutic composition described herein may be a liquid formulation, however lyophilized formulations can also be used. In cases where lyophilized formulations are used, reconstitution is advantageously effected in a buffer at a suitable pH to ensure the viability of the organisms.

The formulation of the probiotic strains in a liquid formulation is highly advantageous. Being under biologically active conditions, the formulation serves also as a supportive medium for living bacteria. As a result, the liquid formulation of the present embodiments, for example, is therapeutically active immediately following oral administration, as no biomass generation in the gut is required.

The liquid formulation of the probiotic *E. coli* strain, typically comprises a suspension of the bacteria in an aqueous solution. The aqueous solution is typically mainly comprised of distilled water, salt in an isotonic amount and can further comprise other ingredients, as is further detailed hereinbelow. More preferably, the carrier comprises 0.6 % saline solution.

A pharmaceutically effective amount of *E. coli*, may range between about 10⁷ and about 10¹² viable bacteria per administration, more preferably between about 10⁸ and about 10¹¹ viable bacteria per administration, more preferably between about 10⁹ and about 10¹¹ viable bacteria per administration and most preferably it is about 5 x 10⁹ viable bacteria per administration.

The term "about", as used herein throughout, refers to ± 10 %.

Techniques for formulation and administration of drugs may be found in "Remington's Pharmaceutical Sciences," Mack Publishing Co., Easton, PA, latest edition, which is incorporated herein by reference.

Biotherapeutic compositions of the present embodiments may be manufactured by processes well known in the art, e.g., by means of conventional mixing, dissolving, granulating, dragee-making, levigating, emulsifying, encapsulating, entrapping or lyophilizing processes.

Biotherapeutic compositions for use in accordance with the present embodiments thus may be formulated in conventional manner using one or more pharmaceutically acceptable carriers comprising excipients and auxiliaries, which facilitate processing of the active ingredients into preparations which can be used pharmaceutically.

For oral administration, the *E. coli* strains and the antibiotics can be formulated readily by combining with pharmaceutically acceptable carriers as described herein. Such carriers enable the active ingredients to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active ingredient doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

It will be appreciated that the compositions of the present embodiments can be encapsulated into an enterically-coated, time-released capsule or tablet. The enteric coating allows the capsule/tablet to remain intact (i.e., undissolved) as it passes through the gastrointestinal tract, until such time as it reaches the small intestine.

Methods of encapsulating live bacterial cells are well known in the art (see, e.g., U.S. Patents to General Mills Inc. such as U.S. Pat. No. 6,723,358). For example, micro-encapsulation with alginate and Hi-MaizeTM starch followed by freeze-drying has been proved successful in prolonging shelf-life of bacterial cells in dairy products [see, e.g., Kailasapathy et al. Curr Issues Intest Microbiol. 2002 Sep;3(2):39-48]. Alternatively, entrapment of viable probiotic in sesame oil emulsions may also be used [see, e.g., Hou et al. J. Dairy Sci. 86:424-428].

Alternatively, the *E. coli* strains may be in dry, powder form, for constitution with a suitable vehicle, e.g., sterile, pyrogen-free water or saline, before use.

The liquid formulation used in context of the present embodiments is preferably orally administered and as such, it preferably further comprises one or more flavoring agent(s).

The flavoring agent can be any known Food grade additive, such as, for example, chocolate fudge flavor (available from Noville Essential Oil Col., North Bergen, N.J. 07047) and Base Strawberry (Cat. No. 10333-33,v Givaudan Dubendorf Ltd., Dubendorf, Switzerland CH-8600), and any other flavoring agents approved by the Fragrance Institute or any other regulatory authority. The flavoring agent can optionally be a sweetener such as, but not limited to, sucrose, corn syrup, saccharin and aspartame.

A representative example of a liquid formulation of a probiotic *E. coli* strain and an antibiotic includes a suspension of the probiotic bacteria in a distilled-water solution that comprises 0.6 % sodium chloride (saline) and 0.1 % flavoring agent such as Base Strawberry. It will be appreciated that the sodium chloride is primarily used for maintaining the liquid in the formulation isotonic to the bacteria cells and hence can be replaced by isotonically equivalent amounts of other highly water soluble salts.

The liquid formulation of the present invention may optionally further comprise one or more volatile fraction(s) of a plant extract, as well as salts. A preferred volatile fraction, according to the present invention, is prepare by first obtaining a water extraction of plant material and thereafter steam distilling the plant extract at a pressure lower than atmospheric pressure and at a temperature that does not exceed 38 °C. A detailed description of such volatile fractions and the preparation thereof is found in WO 02/43649.

The use of volatile fractions of plant extracts prepared as described hereinabove within the liquid formulation of the present invention is particularly advantageous as these volatile fractions are known to maintain the viability of microorganism for a prolonged period of time, at room temperature and/or when refrigerated. Hence, liquid formulations that comprise a probiotic *E. coli* strain and a volatile fraction of a plant extract can be stored for long time periods under standard conditions and therefore have a long shelf-life. The volatile fractions can further serve as flavoring agents. In addition, it is shown in WO 02/43649 that the volatile fractions described herein have therapeutic activity in themselves with respect to GI disorders.

The composition herein described may optionally further comprise one or more additional active ingredients, which may have beneficial therapeutic effects, such as, for example, an anti-inflammatory drug, an immunomodulator, an antibiotics, an anti-diarrheal, a laxative, a pain reliever, an iron supplement, or an Additional probiotic strain. Exemplary active ingredients that can be beneficially used in this context of the present embodiments include, without limitation, non-steroidal anti-inflammatory agents, steroidal anti-inflammatory agents such as corticosteroids, anti-diarrheals, laxatives, pain relievers, iron supplements, additional probiotics, and immunosuppressive medications, with additional probiotics and anti-diarrheals, being preferred.

Non-limiting exemples of suitable anti-inflammatory agents include piroxicam, isoxicam, tenoxicam, sudoxicam, CP-14,304, a salicylate (such as sulfasalazine, olsalazine, mesalamine aspirin, disalcid, benorylate, trilisate, safapryn, solprin, diflunisal, and fendosal), an acetic acid derivative (such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac), a fenamate (such as mefenamic, meclofenamic, flufenamic, niflumic, and tolfenamic acid), a propionic acid derivative (such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic), and a pyrazole (such as phenylbutazone, oxyphenbutazone, feprazone, azapropazone, and trimethazone).

Examples of suitable corticosteroids include, without limitation, hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof.

Examples of suitable additional probiotics include, without limititation, *Bifidobacteria* (such as *B. bifidum*, *B. longum, B. infantis*, *B. breve*, *B. adolescentis*), *Lactobacillus* (such as *L. acidophilus*, *L. plantarum*, *L. casei*, *L. salivarius*, *L. brevis*, *L. fermentum*, *L. helveticus*, *L. delbruekii)*, *Lactococcus*, *Saccharomyces, Streptococcus thermophilus*. *Enterococcus*, *Escherichia coli*, *Pediococcus acidilactici*, *Propionibacterium freudenreichii.*

Examples of suitable anti-diarrheal agents include, for example, anti-motility agents, which slow the passage of stool through the intestine (such as lopermide, diphenoxylate hydrochloride, and difenoxin hydrochloride); adsorbents (such as attapulgite, calcium polycarbophil); and anti-secretory agents, which decrease the secretion of fluid into the intestine, such as bismuth subsalicylate.

Examples of suitable laxatives, include, for example, osmotic laxatives (such as magnesium citrate, magnesium oxide, magnesium hydroxide, and sodium phosphate); bulk-forming laxatives (such as psyllium husk, methylcellulose and polycarbophil), lubricant laxatives, such as mineral oil; stool softeners, such as docusate; and stimulant laxatives (such as bisacodyl, senna, and casanthranol).

Examples of suitable pain relievers include, for example, a non-steroidal inflammatory agent (such as piroxicam, isoxicam, tenoxicam, sudoxicam, and CP-14,304; salicylates, such as aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone); or a narcotic (such as morphine, codeine, thebaine, diamorphine, tramadol, buprenorphine, pethidine, oxycodone, hydrocodone, diamorphine, hydromorphone, nicomorphine, methadone, levomethadyl acetate hydrochloride, pethidine, fentanyl, alfentanil, sufentanil, remifentanil, ketobemidone, carfentanyl, propoxyphene, dextropropoxyphene, bezitramide, piritramide, pentzocine, phenazocine, buprenorphine, butorphanol, nalbufine, dexocine, etorphine, tilidine, tramadol, loperamide, and dipheoxylate).

The amount of a composition to be administered will, of course, be dependent on the subject being treated, the severity of the affliction, the manner of administration, the judgment of the prescribing physician, etc.

The biotherapeutic compositions described herein are preferably presented in a packaging material, such as a FDA approved kit, which may contain one or more unit dosage forms containing the active ingredient. The packaging material may, for example, comprise metal or plastic foil, such as a blister pack.

The biotherapeutic compositions are identified in print, on or in the packaging material, for use in the treatment or prevention of a condition caused by an anaerobic bacterium. The packaging material may be accompanied by instructions for administration. The packaging material may also be accompanied by a notice associated with the conteiner in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription and non-prescription drugs or of an approved product insert.

The composition of the present embodiments is particularly effective for the treatment and prevention. The disorders caused by anaerobic bacteria as referred to in claims 1 - 9. As described in Example 3, and demonstrated in Table 7 below, a synergistic effect was obtained with combined administration of metronidazole and M-17, which was significantly higher than the effects of either treatment alone.

Hence according to another aspect of the present invention, there is provided the use of a pharmaceutically effective amount of a probiotic Escherichia coli strain as described herein, and a pharmaceutically effective amount of at least one anaerobic bacteria antibiotic to which said Escherichia coli strain is resistant, as described herein, in the manufacture of a medicament for treating or preventing a condition caused by an anaerobic bacterium as characterised by the appended claims.

The anaerobic bacteria antibiotic and the probiotic strain may be administered as individual formulations, either simultaneously or sequentially wherein the antibiotic is administered prior to, concomitant with or subsequent to the probiotics *Escherichia coli* strain.

The anaerobic bacteria antibiotic and the probiotic strain together form a part of a biotherapeutic composition, as described herein.

Conditions caused by an anaerobic bacterium which are treatable by the compositions and uses of the present invention include intestinal disorders, such as, for example, pouchitis, microbial infection, gastroenteritis, irritable bowel syndrome, inflammatory bowel disorder (such as Crohn's disease, ulcerative colitis, indeterminate colitis, microscopic colitis and collagenous colitis), other types of colitis (including mucous colitis, granulomatous, ischemic, radiation, or infectious colitis), diarrhea, constipation, colorectal cancer, peritonitis, intra-abdominal abscesses, irritable bowel syndrome, small bowel bacterial overgrowth, duodenitis, jejunitis, proctitis and liver abscess.

The compositions and methods presented herein are particularly useful in the treatment of pouchitis, which is an inflammation in the reservoir made of the distal small bowel during colectomy surgery.

As described in detail in Background section hereinabove, the cause of pouchitis is unknown, but has been linked to low levels of bacteria normally found in the intestine, and to colonization of the pouch by sulfate-reducing bacteria. Hence, pouchitis and related conditions may be treatable by anaerobic bacteria antibiotics.

Conflicting reports have been published regarding the efficacy of metronidazole in the treatment of pouchitis. Based on the observation that some patients do not respond to metronidazole, it has been suggested that there are at least two forms of pouchitis: a bacteriological one that responds to antibiotics, and an antibiotic-refractive form, which requires other medications.

The actual mechanism of action of metronidazole is uncertain. It has been suggested (*Levin*, *1992*) that metronidazole affects pouchitis not by an antibacterial action, but rather by its capacity to remove oxygen radicals, or due to immunosuppressive activity.

The use of metronidazole and a probiotic in the treatment of all forms of pouchitis has not been previously disclosed.

Well-established animal models of pouchitis are currently lacking (*Chen*, *2002*). Hence, probiotics have typically been tested for efficacy in animal models of IBD. Animal models for experimental intestinal inflammation can be classified into spontaneous and induced models, all of which are used to study acute and chronic inflammation. The most widely used models are induced by administering toxic chemicals such as acetic acid, formalin, indomethacin, trinitrobenzene sulfonic acid, or polysaccharides such as dextran sulfate sodium, carrageenan, or immune complexes. In toxic chemical models, acute colonic injury is induced after intracolonic administration of substances that are able to produce colonic epithelial injury followed by rapid influx of granulocytes and monocytes or macrophages, defining the classic features of acute intestinal inflammation.

In order to assess the efficacy of various treatments for ulcerative colitis (UC) and pouchitis in an animal model, the dextran sulfate sodium (DSS) model (*Okayasu*, *1990*) was selected as an animal model for an inflammatory bowel disease. Experimental DSS colitis in Sprague-Dawley rats has been shown to be highly reproducible, and to share most features with human ulcerative colitis, not only from a structural and clinical, but also from an ultrastructural point of view (*Gaudio*, *1999*). The DSS-induced colitis of rodents is characterized by initial acute colonic injury, followed by a slow colonic regeneration and concomitant chronic colitis after stopping the administration of DSS in drinking water. The active form of this model is predated by non-inflammatory epithelial damage, probably as a consequence of a direct toxic effect of DSS on colonic epithelial cells, a pathogenic feature hypothetically shared also by UC.

Mice that developed acute colitis showed signs of diarrhea, gross rectal bleeding and weight loss. On postmortem examination, multiple erosions and inflammatory changes including crypt abscesses were found on the left side of the large intestine. Acute-phase cell infiltrates are confined to the lamina propria, and injury is limited to the mucosa and lamina propria.

Mice that developed chronic colitis showed signs of erosions, prominent regeneration of the colonic mucosa, including dysplasia, shortening of the large intestine, and frequent formation of lymphoid follicles. The population of intestinal microflora *Bacteroides distasonis* and *Clostridium* increased significantly in mice with acute and chronic ulcerative colitis. Morphological studies suggested that the administered DSS was partially phagocytized by macrophages in the colonic mucosa. This model is therefore considered to be adequate to verify the efficacy of drugs to prevent or heal colic lesions.

Preliminary studies by the present inventors suggested that the administration of 2 % DSS to C57BL/6 mice for 6 days produces colitis, without significant mortality. Several probiotic agents have previously demonstrated some efficacy in this model. These include *Lactobacillus* and *Bifidobacterium* which were shown to cause both bacterial translocation to the mesenteric lymph nodes and Enterobacteriaceae bacterial translocation to the liver, in treatment groups as compared to a colitis control. A positive correlation has been established between the severity of colonic inflammation and the extent of bacterial translocation (*Araki*, *2004*; *Osman*, *2004*; *Araki*, *2000*). The efficacy of probiotics in infectious colitis and antibiotic-associated diarrhea has been shown in different clinical trials and also in preventing colitis in IL-10-deficient mice.

Using the acute DSS-induced colitis model, the efficacy of the *Escherichia coli* strain M-17 and of the antibiotic metronidazole, as well as combinations of metronidazole and M-17, was studied, as shown in Examples 1 to 9 in the Examples section that follows.

These Examples investigated the effects of M-17 and metronidazole, as well as combinations thereof, on various parameters of acute DSS-induced colitis, including a clinical marker (DAI), morphological markers (colon length and segmental colon weight), and biochemical markers (MPO, and pro-inflammatory cytokines).

Measurement of Disease Activity Indices (DAIs) provides comprehensive functional measures that are analogous to clinical symptoms observed in human ulcerative colitis, and a significant decrease in the DAI is considered an endpoint of a successful therapy. Measurement of DAIs includes the assessment of changes in body weight, stool consistency changes, and the presence of occult fecal blood/gross rectal bleeding. A four-point system was employed (see, Table 1 hereinbelow). Total colon length is used as a morphometric parameter of colonic injury (*Okayasu*, *1990*; *Gaudio*, *1999*; *Egger*, *2000*). Colonic MPO is used as a biochemical marker of neutrophil influx into the colonic lumen (*Fitzpatrick*, *2000*).

The dose related effects of M-17 on DSS-induced colitis in mice was first studied (see, Example 1 in the Examples section that follows), using three different concentrations of M-17.

During days 1 to 7 of the study, there were only slight increases in the mean DAIs, as well as only slight decreases in the mean body weights of probiotic treated mice, as compared to the control group (see, Tables 3 and 4 hereinbelow). These data demonstrate that the mice could tolerate M-17, when administered in this dose range.

As shown in Table 3 hereinbelow, during the DSS phase (days 7 to 13), DAIs progressively increased in the saline/DSS treatment group. As further shown in Table 3, following induction of colitis, a decrease in DAIs was shown in mice treated with M-17 as compared to colitis-induced animals receiving only saline. The reduction in DAIs as compared to the control group was greatest at the highest concentration studied (5 x 10⁹ CFU/ml), indicating a dose-related effect of M-17 on colitis.

Similarly, as shown in Table 4, the decrease in percentage initial body weight following induction of colitis was lowest in the group treated with 5 x 10⁹ CFU/ml M-17, further demonstrating the beneficial effect of the probiotic bacterial strain in treatment of colitis.

Furthermore, as shown in Table 5 hereinbelow, DSS-induced colitis is accompanied by a decrease in colon length, and an increase in colon weight, MPO activity, IL-1β level and histology. The changes in these parameters following induction of colitis were decreased by administration of 5 x 10⁹ CFU/ml M-17.

Referring now to Example 2 in the Examples section that follows, the efficacy of M-17 in treatment of colitis was compared to that of the antibiotic metronidazole. As shown in Table 6 hereinbelow, mean DAI scores during the latter portion of the DSS phase (days 12 and 13) were significantly lower in mice that received M-17, metronidazole, or a combination of these agents when compared to mice that received vehicle. In this regard, probiotic-, antibiotic-, and probiotic plus antibiotic-treated mice had reduced occurrences of loose and bloody stools. There was only a 40 % incidence of such symptoms in mice treated with the probiotic plus antibiotic, while this incidence was 100 % in vehicle-treated controls.

Referring now to Example 3 in the Examples section that follows, the combined effects of M-17 and metronidazole in the treatment of colitis were studied. A reduction in colon length is a consistent finding in DSS-treated animals and is often used as a marker of colonic injury (*Fitzpatrick*, *2000*; *Gaudio*, *1999*). As shown in Table 7, the mean colon lengths of the probiotic- and antibiotic-treated mice were all longer than in vehicle-treated mice. Taken as a whole, the DAI and colon length data suggest that both symptomatic and gross morphological parameters of colitis were significantly improved in the antibiotic and probiotic treatment groups, both individually and moreover when administered in combination. As further shown in Table 7 therein, treatment with either M-17 or metronidazole attenuated the up-regulation of pro-inflammatory cytokines (IL-12, IL-6, IL-1β, IFN-γ) within the colons of DSS-treated mice. Many of these probiotic-mediated reductions in colonic cytokine levels attained statistical significance (P < 0.05) as compared to the saline-treated control group. Overall, the efficacy profile of M-17 was generally similar to that of metronidazole. The reduction in pro-inflammatory cytokine levels were greater with combined M-17 and metronidazole treatment. In this regard, the combination therapy regimen completely normalized the colonic level of IFN-γ.

The colonic level of IL-10 was decreased in saline treated mice given DSS for a 10 day period. It is suggested that this attenuation of IL-10 may contribute to the development of colitis, since IL-10 is a well-known immunomodulatory cytokine. Probiotic treatment (alone or in combination) did not tend to normalize colonic levels of the regulatory cytokine, IL-10.

In conjunction with the reduced cytokine levels, other parameters of colitis (colon weight, MPO activity and colonic histology score) were significantly improved (P < 0.05) in the groups of mice that received the M-17 and/or metronidazole. Again, the most prominent effects were seen with the combined treatment regimen.

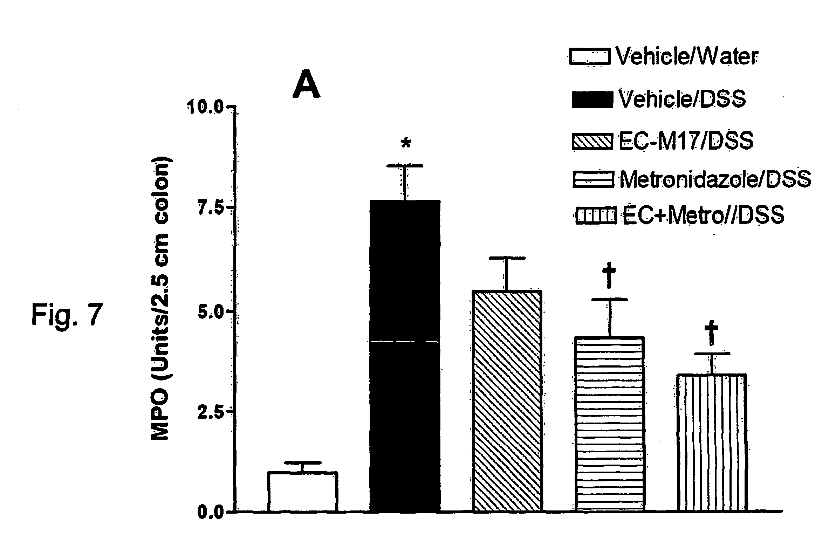

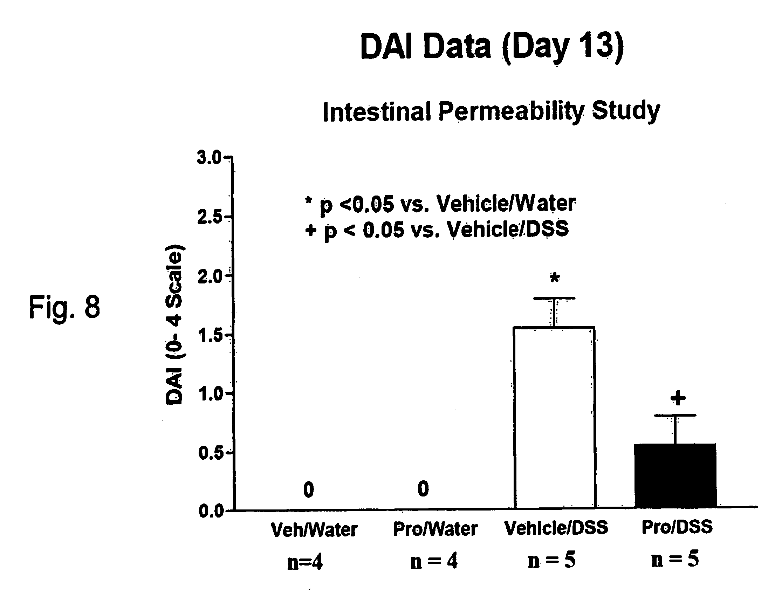

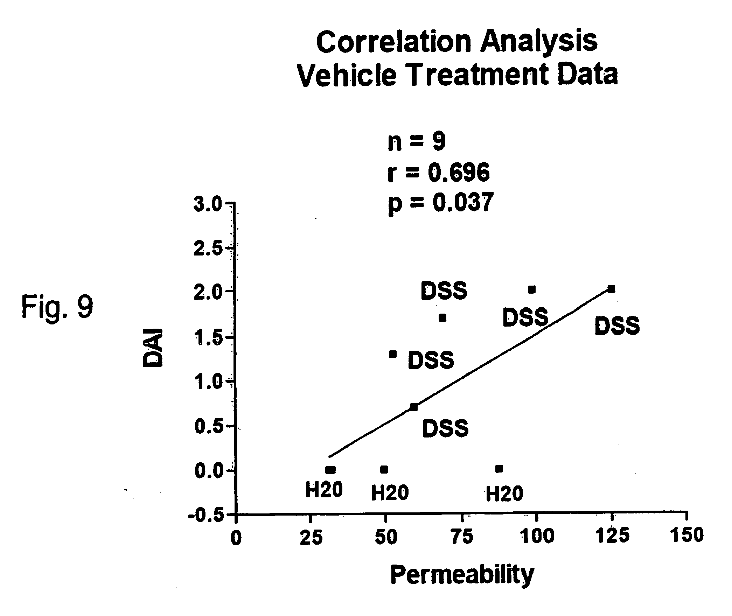

The effect of M-17 on intestinal permeability was investigated, as described in Example 4 in the Examples section that follows. Increased intestinal permeability in Saline/DSS treated animals, as compared to Saline/Water treated control mice have been previously reported (*Kitajima*, *1999*). Similar changes were observed in these studies, as shown in Figure 7. Increased DAIs were observed in DSS-treated mice, as shown in Figure 8, which were greatly reduced by treatment with M-17. The DSS-induced increase in permeability was associated with increased DAIs in saline treated animals. As shown in Figure 9, there was a significant correlation between these two parameters (r = 0.696, p = 0.037. In contrast, there was no significant correlation (i.e., r = -0.484) between intestinal permeability and DAI parameters in M-17-treated mice.

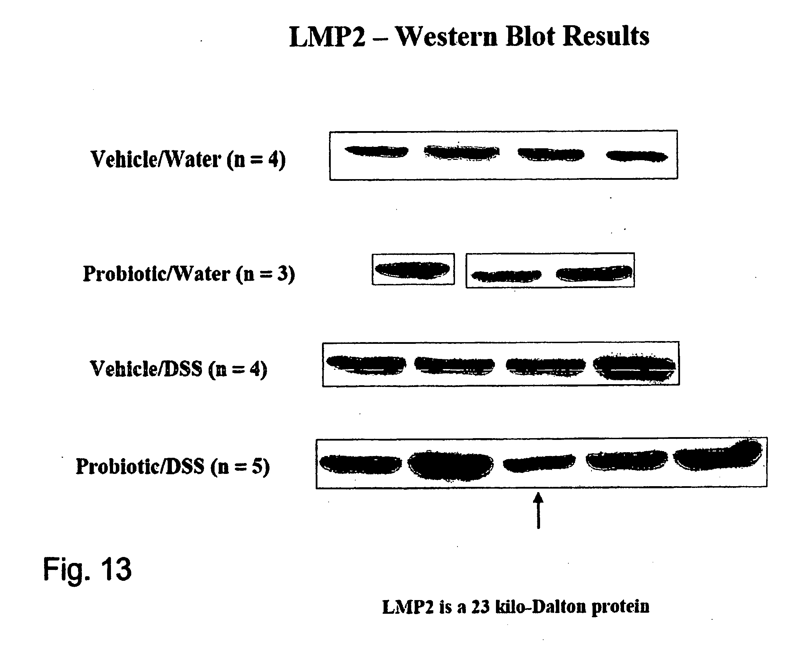

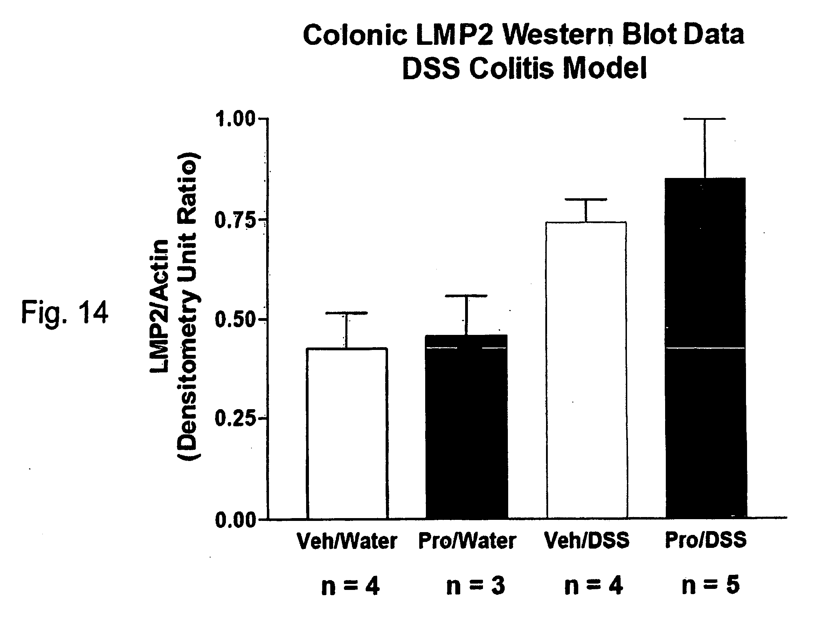

Example 5 investigated the effects of M-17 on Low Molecular Mass Polypeptide-2 (LMP2) expression. It has been previously shown that colonic LMP2 expression is up-regulated in the colons of DSS-treated mice (*Fitzpatrick*, *2004*). This up-regulation of LMP2, as compared to the LMP2 expression in water treated mice, was confirmed in the present study (see, Figures 13 and 14). A single animal that was treated with M-17 (blot marked with an arrow in Figure 13) showed a clear decrease in the colonic LMP2 level. However, M-17 treatment did not affect the expression of colonic LMP2 of the test group in general, since the remaining M-17-treated mice showed no evidence of attenuated colonic LMP2 expression. The densitometry data (Figure 14) was standardized to actin levels; in order to further confirm equal protein loading, during the western blot procedure.

As shown in Example 6 in the Examples section that follows, sensitivity of *Escherichia coli* strain BU-230-98 ATCC Deposit No. 202226 (DSM 12799) to metronidazole was studied. The results obtained demonstrate that this strain is resistant to metronidazole.

The above results demonstrate that mean DAI increases upon administration of 2 % DSS to C57/BL6 mice for a six day period. This DSS-induced increase in DAI was consistently reduced in mice receiving M-17 for a 13 day period. The decrease was greater upon co-administration of M-17 and metronidazole (see, for example, Table 7 hereinunder), indicating that such a combination would be highly effective in the treatment of colitis and related conditions, such as pouchitis. It was further demonstrated that at the concentration of M-17 used in these studies (5 x 10⁹ CFU/ml), the probiotic alone did not induce symptoms of colitis (see, Tables 3 and 6 hereinunder and Figure 8).

As shown in the Examples, the mean colon length was longer in mice receiving M-17 for a 13-day period. These data indicate a beneficial effect of this probiotic in the DSS colitis model. It was also shown that the segmental colon weight tended to increase in mice receiving DSS, which may be at least partially explained by the submucosal edema which is evident with this colitis model (*Okayasu*, *1990*; *Gaudio*, *1999*).

In some studies, the increase in colon weight was partially normalized in mice that received the probiotic strain. Interestingly, in mice receiving both M-17 and metronidazole, the DSS-induced change in colon weight was substantially normalized (see, Table 7), which further indicates the high efficacy and synergistic effect of such a combined treatment.

Studies of colonic MPO indicated that a 5 x 10⁹ CFU/ml dose of M-17 attenuated colonic MPO to some degree in two of the three studies performed (see, Tables 5 and 7). In mice receiving both M-17 and metronidazole, the mean MPO level was more profoundly reduced as compared to animals receiving either the probiotic or the antibiotic alone (see, Table 7).

As shown most clearly in Table 7, treatment with M-17 attenuated the up-regulation of pro-inflammatory cytokines (IL-12, IL-6, IL-1β, IFN-γ) within the colons of DSS-treated mice. These pro-inflammatory cytokines contribute to the pathogenesis of DSS-induced colitis (*Egger*, *2000*; *Fitzpatrick*, *2000*). Greater inhibition of pro-inflammatory cytokine levels was observed with combined M-17 and metronidazole treatment. However, probiotic treatment (alone or in combination) did not significantly modulate colonic levels of the regulatory cytokine, IL-10 (Table 7). The level of colonic IL-1β was attenuated to some degree in mice receiving M-17 in all Examples studied (see, Tables 5-7).

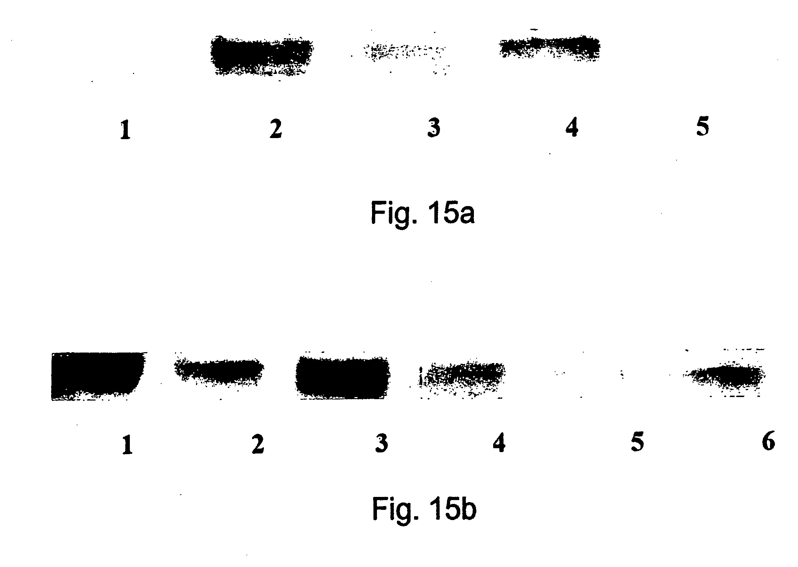

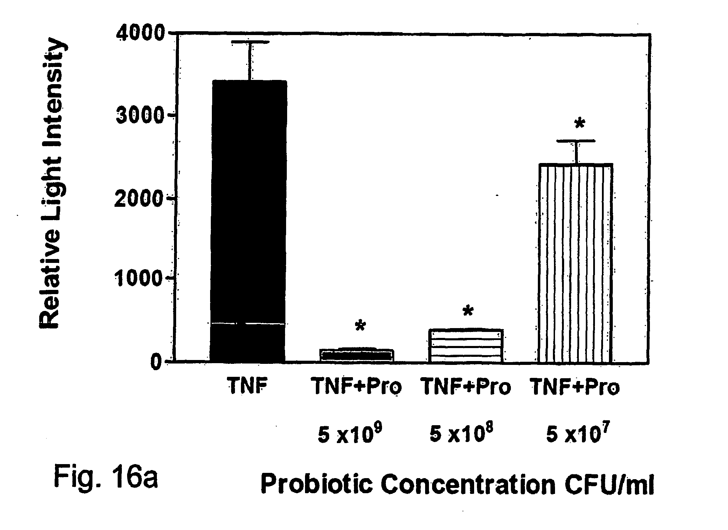

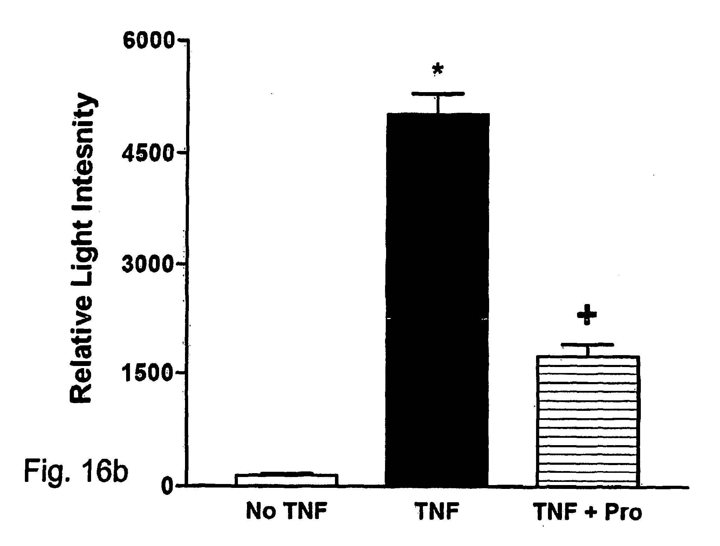

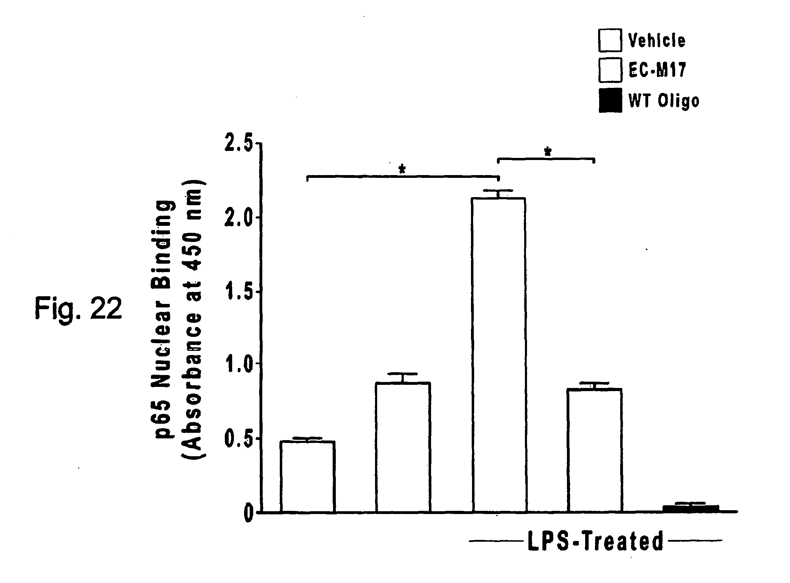

Since production of these pro-inflammatory cytokines (TNF-α, IL-1β, and IL-6) is dependent on activation of NF-κB, the effects of M-17 on the NF-κB signaling system was also examined in the DSS-induced colitis model. Interestingly, the administration of M-17 to DSS-treated mice resulted in attenuated nuclear expression of the NF-κB p65 subunit (see, Figure 15). The nuclear expression of the p65 has previously been shown to be up-regulated during DSS-induced colitis, and is thought to play a critical role in promoting intestinal inflammation (*Murano*, *2000*; *Spiik*, *2002).* For instance, p65 activates the transcription of SMAD7, which blocks the inhibitory effect of TGF-β on intestinal inflammation, thereby promoting IBD (*Monteleone*, *2001*). Without being bound by any particular theory, these results suggest that reductions in the parameters of DSS-induced colitis, including pro-inflammatory cytokine levels, may have resulted from the inhibition of NF-κB signaling by M-17. Thus, the obtained *in vitro* data showed that M-17 inhibited the nuclear binding of NF-κB in murine macrophages (see, Figure 22) and in a reporter gene assay conducted in embryonic kidney cells (see, Figure 16).

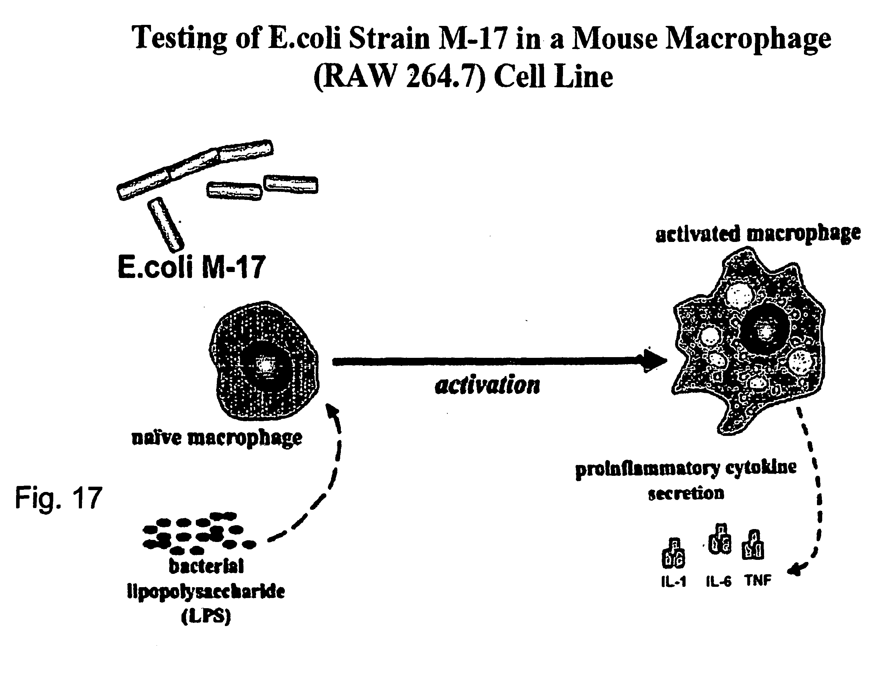

In order to further study the effect of *E. coli* strains on pro-inflammatory cytokines, attenuation of cytokine levels of IL-1β and TNF-α was studied *in vitro*, as described in Example 11 below, and represented schematically in Figure 17. A large number of different *E. coli* strains were tested for attenuation of cytokine production by LPS-activated macrophages.

LPS is thought to bind to the toll-like receptor 4 isoform B (TLR4), which is a critical component of the heteromeric receptor complex that transduces signals delivered by LPS of Gram-negative bacteria, and cause downstream signaling through NF-κB and mitogen-activated protein (MAP) kinase pathways. This leads to cytokine secretion (IL-1, TNF, IL-6).

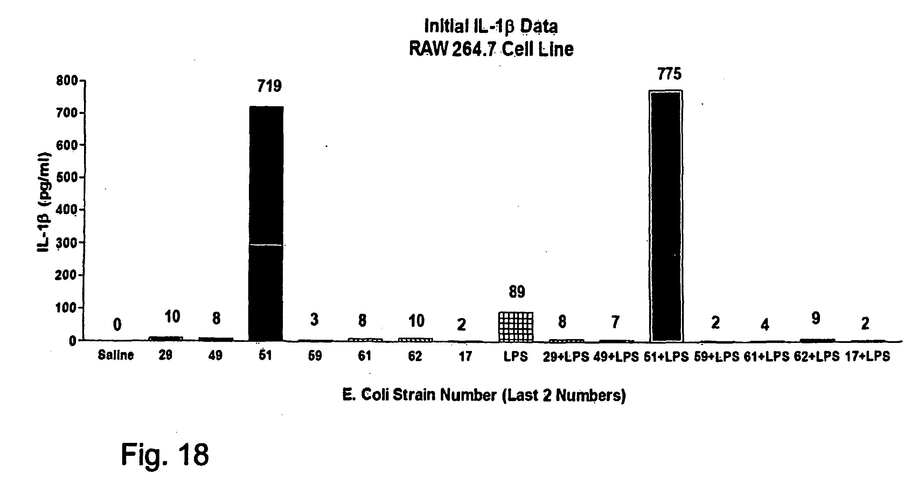

As shown in Figure 18, all strains except for ECOR-51 inhibited LPS-induced IL-1β secretion, with the highest levels of inhibition shown by M-17 and ECOR-59.

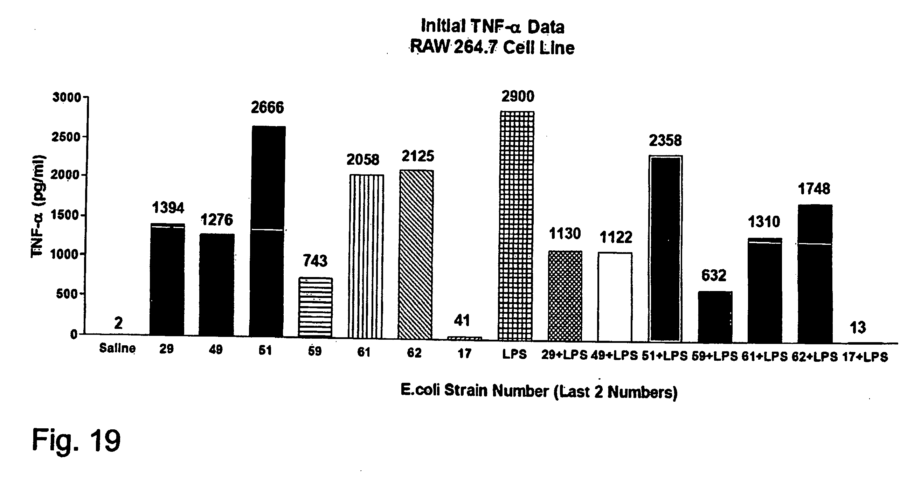

As shown in Figure 19, it was surprisingly found that M-17 was uniquely effective in fully inhibiting the level of TNF-α produced by LPS-activated macrophages. All other strains tested in these studies showed only partial inhibition.

Since, as discussed in detail in the Background section hereinabove, pro-inflammatory cytokines, which include interleukin-1β, TNF-α, IL-8 and IL-12 appear to play an important role in diseases such as pouchitis and ulcerative colitis, these results suggest that M-17 exerts its beneficial effect via inhibition of secretion of these cytokines.

As described in Example 8, modulation of aerobic and facultative bacterial flora was monitored. DSS administration reduced the dominant aerobic *Pseudomonas* population, indicating that this opportunistic pathogen has little, if any, direct role in progression of the disease. M-17 was not shown to be a major component of the facultative intestinal flora. Overall, in mice treated with M-17 plus metronidazole, there was no significant change in the total proportions of mice having detectable fecal *E. coli* as compared to saline/DSS treated mice. It is therefore suggested that the major anti-colitic action of M-17 may be due to modulation of immune processes, rather than alteration of intestinal microflora or competitive exclusion of endogenous bacteria. This suggestion is consistent with the results obtained for NF-κB and pro-inflammatory cytokine levels. The combined effects of M-17 and metronidazole on parameters of DSS-induced colitis indicate separate mechanisms of action of the two therapeutic agents. In this regard, the attenuation of colitis parameters by metronidazole indicates that an anaerobic bacterium such as *Bacteroides* or *Helicobacter* may contribute to the pathogenesis of murine colitis.

In summary, *in vitro* treatment of two cell lines with live M-17 resulted in inhibition of the NF-κB signaling pathway and p65 nuclear binding. Moreover, the M-17 probiotic attenuated the secretion of pro-inflammatory cytokines by macrophages. In *in vivo* studies, it has been shown that M-17 effectively improved various histological, biochemical, morphological, and symptomatic parameters of DSS-induced colitis in mice. Combined treatment with M-17 plus metronidazole proved to be generally more effective in DSS-induced colitis in mice than either alone. These data suggest that M-17 could prove to be clinically useful for the treatment of intestinal inflammatory diseases.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following example, which together with the above descriptions, illustrate the invention in a non limiting fashion.

### MATERIALS AND EXPERIMENTAL METHODS

### Chemicals:

Dextran Sulfate Sodium Salt (DSS), MW 36,000-50,000 (lot numbers 2387F, 5464H and 7904H) was purchased from MP Biomedicals (Aurora, OH).

Sterile 0.9 % sodium chloride injection bottles (lot number 26-451-K) were obtained from Hospira Inc., (Lake Forest, IL). Sterile water injection bottles (lot numbers 20-239-DK and 23-409-DK) were purchased from Abbot Laboratories (North Chicago, IL). Ultra-pure distilled water (DNAse, RNAse free, Lot # 1271392) was obtained from GIBCO (Grand Island, NY).

3,3',5,5'-tetramethylbenzidine {TMB}, N,N-dimethylformamide {DMF}, hydrogen peroxide, and hexadecyltrimethylamoniun bromide {HTAB}) were purchased from Sigma Chemical Company (Saint Louis, Mo).

Formamide and Evans Blue were purchased from Sigma.

RC-DC Protein Assay was purchased from Bio-Rad (Hercules, CA).

Luminol reagent (Western Lightning) was purchased from Perkin-Elmer Life Sciences Inc. (Boston, MA).

Rabbit polyclonal antibody to Low Molecular Mass Polypeptide-2 (LMP2) was purchased from Research Diagnostics Inc. (Flanders, NJ).

Mouse cytokine ELISA kits (IL-1β, IL-6, IL-10, IL-4) were obtained from Pierce Endogen Inc. (Rockford, IL). The IL-12 mouse ELISA kit was purchased from Biosource International (Camarillo, CA). The mouse MIP-2 ELISA kit was obtained from R&D Systems (Minneapolis, MN).

### Animals:

Male C57 BL/6 mice were obtained from The Jackson Laboratory (Bar Harbor, Maine). All mice were obtained at 8 to 10 weeks of age. Mice were housed in single cages at the animal research facility of the Penn State College of Medicine (Hershey, PA), having a 12 hour light-dark cycle, with the light phase between 7 a.m. and 7 p.m.

Mice were provided with standard pelleted chow, ad libitum, and with filtered water, as described below.

### Preparation of Probiotic strain, Metronidazole and Dosing Procedure:

*E. coli* strain M-17 was provided by the BioBalance Corporation, as a probiotic suspension of about 1 x 10¹¹ CFU/ml in 0.6 % saline.

A 0.6 % saline solution was prepared by diluting sterile 0.9 % saline with sterile water. Subsequently, the 1 x 10¹¹ CFU/ml solution of saline was typically diluted 20-fold in 0.6 % saline, to obtain an *E. coli* strain M-17 concentration of 5 x 10⁹ CFU/ml.

Metronidazole (SigmUltra, Cat. No. M1547) was suspended in 0.6 % saline, then heated with hot water for approximately 2 minutes until dissolved.

Mice were dosed once daily for a period of 13 days, by oro-gastric gavage with a 20 gauge, one-inch long needle (Popper & Sons, New Hyde Park, NY). Dosages comprised either 40 mg/kg dose of metronidazole or 5 x 10⁹ CFU/ml probiotic solution. A 0.6 % saline solution was administered as a control.

Disease Activity Indices (DAIs) were determined either daily or on alternate days, by evaluation of stool consistency and occult blood in the stool and weight loss on a standardized severity scale, as shown in Table 1 (*Murthy*, *1993*).

**Table 1: DAI Scoring System**

| Score | Weight Loss | Stool Consistency | Occult Blood/Gross Bleeding |
|---|---|---|---|
| 0 | None | Normal | Negative |
| 1 | 1-5% | Loose Stool | Negative |
| 2 | 5-10% | Loose Stool | Positive |
| 3 | 10-15% | Diarrhea | Positive |
| 4 | >15% | Diarrhea | Gross Bleeding |

Disease Activity Index (DAI) Calculation: The individual scores for weight loss, stool consistency, and occult blood/bleeding are determined, and divided by 3 to determine a mean DAI for the mouse. For example, if an animal lost 12 % of the initial body weight [score =3], had evidence of diarrhea [score = 4], and evidence of gross rectal bleeding [score = 4], the mean DAI would be 3 + 4 + 4 = 11/3= 3.7

In Example 3, weight loss was not very evident in the DSS-treated mice. Therefore, a modified DAI calculation was used. The modified DAIs were calculated similarly to the DAIs of Table 1, but only the stool consistency and occult blood components were used for this determination.

### Preparation of Drinking Water and DSS solution:

Tap water was filtered with a 0.22 micron filter system (Millipore Corporation, Billerica, MA), to remove endogenous microbes. 2 % DSS (w/v) was prepared by dissolving in the filtered water.

### DSS-Induced Colitis:

In order to study the effects of *E*. *coli* strain M-17, C57BL/6 mice were first provided with filtered water containing no DSS for days 0 to 7 of the study. During the initial 7-day period, mice were dosed once daily with 0.6 % saline, M-17 (5 x 10⁹ CFU/ml), metronidazole (40 mg/kg), or both M-17 (5 x 10⁹ CFU/ml) and metronidazole (40 mg/kg). Colitis was then induced by providing the mice with a solution of 2 % DSS (w/v) dissolved in the filtered water for days 7 to 13 of the study. One group of mice received water without DSS. Water consumption and body weight were recorded throughout the study.

During this time period, DAIs were determined, as described above.

### Measurement of Colonic Myeloperoxidase (MPO):

On day 13 of the study, animals were euthanized by exposure to carbon dioxide. The colon was rapidly removed and the total colon length was determined to the nearest 0.1 cm. Typically, a 2.5 cm segment of distal colon was fixed in 10 % buffered formalin, for a subsequent histological evaluation (see below). The adjacent 2.5 cm segment of colon was weighed and then frozen in liquid nitrogen. This segment of colon was used for the evaluation of colonic MPO, as well as colonic cytokine levels.

The 2.5 segment of colon was homogenized for about 30 seconds in 0.5 ml of molecular biology grade distilled water, using a hand-held tissue homogenizer (TissueMiser) from Fisher Scientific (Pittsburgh, PA). The homogenate was then centrifuged at 10,000 RPM at 4° C for 15 minutes. The pellet was resuspended in hexadecyltrimethylammonium bromide (HTAB) buffer (pH 6.0); for the measurement of colonic MPO. MPO was assayed by the tetramethylbenzidine (TMB) method (*Fitzpatrick, 2000).*

### Measurement of Colonic Cytokine Levels:

The supernatant fraction from the colonic homogenate (see above) was aliquoted into appropriate test tubes and frozen at -70 °C. Subsequently, the supernatants were used to measure relevant pro-inflammatory cytokines [e.g., IFN-γ, IL-1β, TNF-α, IL-12] using commercially available mouse ELISA kits (*Fitzpatrick*, *2000).* The data was typically expressed as pg/2.5 cm of colon.

### Protein Determinations:

In Example 5, the whole colon was also used, for the determination of colonic LMP2 levels. Colons were homogenized in lysis buffer at a 1:8 (weight to volume) ratio. Whole cell extracts of colon (20 µg), as determined by the Bio-Rad method, were used in the western blot study for LMP2.

### Assessment of Colonic Histological Damage:

Distal colonic specimens were processed by standard methods and embedded in paraffin by the Histology core facility at the Penn State University College of Medicine. Microscope slides were coded. Using coded hematoxylin-eosin H&E slides (1 slide per mouse) from the distal colon, colonic histology scores were determined from six different areas on the slide (*William*, *2001*; *Krieglstein*, *2001*). The 0 to 40 point scoring system is shown in Table 2 below. A mean total histology score value was calculated for each mouse.

**Table 2: Colonic Histology Scoring System**

| FEATURE SCORED | SCORE | DESCRIPTION |
|---|---|---|
| Inflammation Severity | 0 | None |
| | 1 | Mild |
| | 2 | Moderate |
| | 3 | Severe |
| Inflammation Extent | 0 | None |
| | 1 | Mucosa |
| | 2 | Mucosa and Submucosa |
| | 3 | Transmural |
| Crypt Damage | 0 | None |
| | 1 | 1/3 of crypt damaged |
| | 2 | 2/3 of crypt damaged |
| | 3 | Crypts lost, surface epithelium present |
| | 4 | Crypts and surface epithelium lost |
| Percent Involvement (Multiply for 3 features above) | 0 | 0% |
| | 1 | 1-25% |
| | 2 | 26-50% |
| | 3 | 51-75% |
| | 4 | 75-100% |

Total Histology Scores were determined by multiplying the percent involvement for each of the 3 different histological features by the area of involvement. Using this scoring system, the minimal score = 0 and the maximal score = 40. The percent involvement was determined with a 25 mm ocular grid attached to an Olympus CH light microscope. The histological evaluation was performed at a 400 x magnification.

### Measurement of Colonic Permeability:

The effects of M-17 on intestinal permeability were evaluated by the Evans Blue (EB) dye method (*Kitajima*, *1999*). Briefly, on day 13 of the acute DSS-colitis paradigm, mice were anesthetized with sodium pentobarbital. The colon was washed free of fecal contents and 0.2 ml of 1.5 % (w/v) of EB was injected into a ligated segment of colon. The mice were allowed to recover from surgery and then euthanized after two hours. The colon was removed, weighed and then incubated in formamide. Subsequently, the amount of EB permeating the gut wall was calculated based on the standard curve of EB in formamide.

### Western Blot Study for Low Molecular Mass Polypeptide-2 (LMP2):

Colonic LMP2 was measured as described previously (*Fitzpatrick*, *2004*).
Western blots were performed using a standard technique. Briefly, 4-15 % Tris-HCl ready gels (Bio-Rad) were used for this protocol. The gels were run at 100 volts for about 1 hour. Gels were then transferred onto PROTRAN® nitrocellulose membranes (Whatman/Schleicher & Schuell, Florham Park, NJ). Subsequently, blots were blocked with PBS-Tween containing 5 % blotto (non-fat dried milk). Blots were then incubated in primary antibody (rabbit polyclonal antibody to LMP2), washed in PBS-Tween, and exposed to an appropriate secondary antibody. After a further series of washes, equal amounts of an oxidizing agent and a luminol reagent (Western Lightning) was applied for 1 minute. Subsequently, the blot was dried and exposed to Kodak Scientific Imaging XB-1 film. Relative densitometry analyses were performed on the LMP2 bands with a QuantiScan® software program. Final group comparisons were performed after LMP2 band densities were standardized to band densities obtained from β-actin western blots.

### Microbiological Analysis of Fecal Samples

Coded samples of fecal pellets were cultured on Tryptic Soy Agar, Brain Heart Infusion Agar, Eosin-Methylene Blue (EMB) Agar and Salmonella-Shigella Agar at 37 °C to assess the aerobic and facultative flora of treated and untreated mice. Diluted samples were dispensed and the cultures incubated for 48 hours. The cultures were scored for number of colonies and distinguishing characteristics, such as the metallic green sheen of E. *coli* on medium.

### Nuclear Factor Kappa B (NF-κB) Receptor Gene Assay

The NF-kB reporter stable cell line was obtained from Panomics (Redwood City, CA). This cell line is derived from human 293T embryonic kidney cells, which have an integrated luciferase reporter construct regulated by six copies of the TNF-κB response element. For these studies, the cells were plated into 24-well culture plates and grown to confluence. The medium was then removed and replaced with serum-free medium for 16 hr, before TNF-α was added to the cells. Specifically, the NF-κB signaling pathway was activated, by treating the cells with 100 ng/ml of TNF-α. Typically, the cells were treated with vehicle (0.6% saline), or the M-17 probiotic at a concentration of 1 x 10⁸ CFU ml, immediately prior to TNF-α treatment. All treatments were performed in triplicate. After 6 hours, the cells were washed, and lysed. The amount of luciferase was then quantified using an assay kit from Promega Corporation (Madison, WI) and a Perkin Elmer HTS 7000+ Bioassay plate reader in the luminescence mode.

### Effects of M-17 in a RAW 264.7 macrophages cell line

The RAW 264.7 mouse macrophage cell line was obtained from American Type Culture Collection (ATCC). RAW 264.7 cells were grown in Dulbecco's Modified Eagle's Medium (DMEM) containing 10 % FBS (Fetal Bovine Serum). The lipopolysaccharide (LPS) stimulation studies were conducted at a cell density of 2 x 10⁶/ml. M-17 was added to the macrophage cell culture system, at a concentration of 1 x 10⁸ CFU/ml, just prior to LPS (5 µg/ml). For the NF-κB p65 measurement, nuclear extracts were obtained from cells either immediately (0 hours) or 3 hours after LPS-stimulation. A protein determination of the nuclear extracts was done with the Bio-Rad protein assay (Bio-Rad Laboratories). For the p65 analysis, 10 µg of protein was utilized per sample. The nuclear binding of p65 was measured with the TransAM™ NF-κB p65 assay kit, according to the manufacturer's instructions.

For the cytokine secretion experiments, after 0 or 4 hours of LPS exposure, the culture medium was collected for the measurement of cytokines (TNF-α, IL-1β and IL-6) by ELISA (Pierce-Endogen), according to the manufacturer's directions. Probiotic conditioned culture media (CM) was prepared by adding M-17 to the macrophage culture media 5 minutes or 2 hours. The media was then collected and centrifuged at 10,000 RPM for 10 minutes. Next, the CM was passed through a 0.22 micron filter prior to use in cytokine secretion experiments. In order to heat kill M-17 (HK), the probiotic was boiled at 100° C for 20 minutes. Then, the heat killed M-17 was centrifuged at 10,000 RPM. The cellular pellet was washed in PBS and recentrifuged. The pellet was then resuspended in a sufficient quantity of saline to achieve a final concentration of 1 x 10⁸ CFU/ml. HK and CM were used in cytokine secretion studies with the RAW 264.7 cell line, as described above. CM was used at a final concentration of 10 % (v/v). In some experiments, we also evaluated whether metronidazole (50 µg/ml) could inhibit cytokine secretion.

### Western Blot Analysis of the NF-κB p65 subunit in Murine Colonic Samples:

14 mice were treated, as described above, with Vehicle/Water (n = 2), M-17/Water (n =3), Vehicle/DSS (n =5), or M-17 (n =4). Mice were euthanized on study day 13, and colon samples were snap frozen in liquid nitrogen. A nuclear extract was prepared from colonic homogenates. Protein determination of these colonic extracts was performed using the Bio-Rad protein assay (Bio-Rad Laboratories). For the western blot analysis, 30 micrograms of protein, per colonic sample were used. Western blots were performed using a standard technique. Briefly, 4-15 % Tris-HCl ready gels (Bio-Rad) were used for this protocol. The gels were run at 100 volts for about 1 hour. Gels were then transferred onto PROTRAN® nitrocellulose membranes (Schleicher & Schuell Bioscience Inc). Subsequently, blots were blocked with PBS-Tween containing 5 % blotto (non-fat dried milk). Next, the blots were incubated in primary antibody (rabbit polyclonal antibody to p65), washed in PBS-Tween, and then exposed to an appropriate secondary antibody (goat-anti-rabbit). After another series of washes, equal amounts of an oxidizing agent and a luminol reagent (Western Lightning, Perkin-Elmer Life Sciences Inc.) were applied for 1 minute. Subsequently, the blots were dried and exposed to Kodak Scientific Imaging XB-1 film. NF-κB p65 was expressed as a 65 kilo-dalton protein as determined by internal molecular weight standards (Bio-Rad). For evaluating the levels of colonic LMP2 expression, a densitometry analysis was performed with a QuantiScan software program. The nuclear p65 expression data was standardized to the mean level found in Vehicle/Water treated mice, and it is reported as the fold increase compared to these data.

### Statistical Analysis:

All data were calculated using a GraphPad Prism® (San Diego, CA) computer software program. The values are expressed as the mean ± SEM. The data was normally distributed, as determined by GraphPad Prism. Multiple groups were analyzed by one-way ANOVA, and individual group comparisons by the Newman-Keuls Multiple comparison test. For some data, in order to confirm differences between two treatment groups, the student's t test was utilized (GraphPad Prism). The linear regression analyses were also performed by Graph-Pad Prism. A difference of p< 0.05 was considered significant for all these statistical analyses.

### IN VIVO STUDIES

### EXAMPLE 1

### Dose Related Effects of M-17 on Acute DSS-Induced Colitis in Mice

Three concentrations of M-17 were administered to C57/BL6 mice: low (5 x 10⁷ CFU/ml), medium (5 x 10⁸ CFU/ml) and high (5 x 10⁹ CFU/ml). Control mice received 0.6 % saline. The results are presented in Tables 3-5 below. The effect of M-17 on DSS-induced colitis was measured in terms of the effect on DAIs (Table 3); percentage initial body weight (Table 4); and other parameters of colitis (Table 5).

As shown in Table 3, there was no significant differences in the DAIs of the different treatment groups during the pre-DSS phase (i.e., days 0, 2, 4 and 6). DAIs increased upon induction of colitis. In saline/DSS treated mice, there was a clear increase in the mean DAI value on study days 12 and 13 (Table 3).

As further shown in Table 3, both 5 x 10⁸ CFU/ml and 5 x 10⁹ CFU/ml of M-17 significantly reduced mean DAI scores when compared to vehicle in DSS-treated mice (34.8 %, *P* < 0.05 and 43.5 % *P* < 0.05, respectively). Therefore, 5 mg/kg of a 5 x 10⁹ CFU/ml concentration of M-17 was selected for use in further studies.

**Table 3: DAI Time Course Data - C57/BL6 Mice**

| **Study Day** | **Saline/Water (n =4)** | **Saline/DSS (n = 8)** | **M-17-Low /DSS (n=8)** | **M-17-Med /DSS (n =8)** | **M-17-High /DSS (n = 8)** |
|---|---|---|---|---|---|
| **0** Dose Phase | 0 ± 0 | 0 ± 0 | 0 ± 0 | 0 ± 0 | 0 ± 0 |
| **2** | 0.08 ± 0.08 | 0.08 ± 0.05 | 0.2 ± 0.1 | 0.3 ± 0.2 | 0.2 ± 0.2 |
| **4** | 0.08 ± 0.08 | 0.1 ± 0.1 | 0.3 ± 0.1 | 0.3 ± 0.1 | 0.5 ± 0.2 |
| **6** | 0.2 ± 0.1 | 0.1 ± 0.1 | 0.4 ± 0.1 | 0.3 ± 0.2 | 0.4 ± 0.1 |
| **7** DSS Phase | | | | | |
| **8** | 0 ± 0 | 0.1 ± 0.1 | 0.2 ± 0.1 | 0.4 ± 0.2 | 0.2 ± 0.1 |
| **10** | 0 ± 0 | 0.8 ± 0.2 | 0.5 ± 0.1 | 0.3 ± 0.1 * | 0.4 ± 0.2 |
| **12** | 0 ± 0 | 1.6 ± 0.3 | 1.3 ± 0.2 | 0.8 ± 0.2 * | 1.2 ± 0.4 |
| **13** | 0 ± 0 | 2.3 ± 0.2 | 1.8 ± 0.3 | 1.5 ± 0.2 * | 1.3 ± 0.4 * |

| | | | | | |
|---|---|---|---|---|---|
| * p < 0.05 vs. Saline/DSS on same study day | | | | | |

Weight loss (defined as > 1 %) was noted in about 13 % of saline-treated mice. Similarly, with M-17 treated animals, about 20 % of the mice lost weight. In contrast, weight loss was observed in 50 % of the metronidazole-treated mice, and 90 % of the M-17 + metronidazole treated animals. During the DSS-phase of the study (days 7-13), weight loss was not evident in the saline/water treated group. Some weight loss (defined as > 1 %) was observed in three of the other treatment groups. Specifically, weight loss occurred in 22 % of the saline/DSS treated mice, 10 % of the M-17/DSS treated mice and 30 % of the metronidazole/DSS treated mice. Interestingly, no weight loss was evident in the M-17 + metronidazole group.

**Table 4: Percentage Initial Body Weight Time Course Data - C57/BL6 Mice**

| **Study Day** | **Vehicle/Water (n =4)** | **Vehicle/DSS (n=8)** | **Pro-Low /DSS (n = 8)** | **Pro-Mid /DSS (n =8)** | **Pro-High /DSS (n = 8)** |
|---|---|---|---|---|---|
| **0** **Dose Phase** | 100.0±0 | 100.0 ± 0 | 100.0 ± 0 | 100.0 ± 0 | 100.0 ± 0 |
| **2** | 99.4 ± 1.0 | 101.0 ± 0.9 | 99.8 ± 0.5 | 98.3 ± 0.8 | 97.4 ± 2.2 |
| **4** | 99.1 ± 0.5 | 100.6 ± 0.8 | 98.7 ± 1.2 | 97.7 ± 1.1 | 96.1 ± 2.0 |
| **6** | 99.1 ± 0.5 | 99.6 ± 1.0 | 98.5 ± 1.0 | 98.3 ± 1.1 | 98.7 ± 0.9 |
| **7** **DSS Phase** | | | | | |
| **8** | 101.6 ± 0.7 | 101.7 ± 1.1 | 100.0 ± 1.4 | 98.4 ± 2.0 | 99.2 ± 1.3 |
| **10** | 102.6 ± 1.3 | 101.4 ± 1.1 | 100.1 ± 1.2 | 97.8 ± 1.2 | 97.6 ± 1.6 |
| **12** | 102.2 ± 1.9 | 93.4 ± 1.9 | 93.8 ± 1.0 | 94.7 ± 1.8 | 93.0 ± 2.2 |
| **13** | 103.4 ± 1.9 | 89.0 ± 1.4 | 89.3 ± 1.3 | 89.3 ± 2.4 | 90.3 ± 2.6 |

During the pre-DSS phase of the study, only slight differences in water consumption were observed between treatment groups. Therefore, during this phase of the study, all mice tolerated the antibiotic and probiotic treatment regimes relatively well. Water consumption was generally similar in all treatment groups from days 7 to 12. However, on study day 13, water consumption in the saline/DSS treatment group was significantly decreased than in other treatment groups.

Other parameters of colitis (e.g., colon length, MPO, IL-1β) were improved by treatment with M17 or metronidazole, either alone or in combination, as compared to saline/DSS treated mice (Table 5). All colon lengths in the probiotic and metronidazole treatment groups were significantly longer than those of the vehicle/DSS treatment group.

Interestingly, there was a clear reduction in the mean colonic IL-1β level, when DSS-treated mice also received 5 x 10⁹ CFU/ml of M-17.

**Table 5: DSS Colitis Parameters - Day 13**

| **Parameter** | **Saline/water (n = 4)** | **Saline/DSS (n = 8)** | **M-17-Low /DSS (n = 8)** | **M-17-Med /DSS (n =8)** | **M-17-High /DSS (n = 8)** |
|---|---|---|---|---|---|
| **Colon Length (cm)** | 7.2 ± 0.1 | 5.9 ± 0.2 | 6.4 ± 0.3 | 6.6 ± 0.1 | 6.4 ± 0.3 |
| **Colon Wt. (mg/2.5 cm)** | 66±5 | 99 ± 4 | 111 ± 6 | 102 ± 7 | 84 ± 5 * |
| **MPO (U/2.5 cm)** | 1.4 ± 0.2 | 7.5 ± 0.8 | 7.8 ± 0.6 | 8.2 ± 1.0 | 6.8 ± 1.0 |
| **IL-1β (pg/2.5 cm)** | 5 ± 3 | 680 ± 123 | 1017 ± 207 | 777 ± 131 | 431 ± 112 |
| **Histology (0- 40)** | 5.6 ± 1.9 | 13.2 ± 1.3 | 15.0 ± 2.4 | 14.4 ± 1.8 | 12.3 ± 2.4 |

| | | | | | |
|---|---|---|---|---|---|
| * p < 0.05 vs. Saline/DSS | | | | | |

### EXAMPLE 2

### Comparative effects of M-17, metronidazole and M-17 plus metronidazole in DSS-induced colitis

5 x 10⁹ CFU/ml M-17 or 40 mg/kg metronidazole (Metro) was administered to C57/BL6 mice.

Prior to the administration of DSS (study day 7), there were no significant differences in the DAI scores among the treatment groups (Table 6). Also, during the pre-DSS phase of the study, only slight differences in water consumption were observed among all the treatment groups. Therefore, through study day 6, all mice tolerated the antibiotic and probiotic treatment regimens relatively well and no major overt effects were evident.

The administration of 2 % DSS to otherwise untreated (vehicle/DSS) C57 BL/6 mice resulted in significant increases in DAI scores on days 12 and 13 when compared with vehicle-treated mice that did not receive DSS (vehicle/water; Table 6). However, the increased DAI that was found in vehicle-treated mice was less prominent in mice treated with M-17, metronidazole, and M-17 plus metronidazole. On day 13, the DSS-treated group that also received M-17 (M-17/DSS) had a mean DAI score 68.4 % less, when compared with DSS-treated mice that also received vehicle (*P* < 0.01). DSS-treated mice that received metronidazole (Metro/DSS) also had significantly reduced DAIs (*P* < 0.01), but the reduction was not as large (47.4 %). DSS-treated mice that received both M-17 and metronidazole (M-17 plus Metro/DSS) had significantly reduced DAIs which was reflected in a more profound reduction in the mean DAI score (78.9 %, *P* < 0.01). As on day 13, treatment with M-17, metronidazole, and M-17 plus metronidazole significantly reduced (P < 0.01) the DAI as compared to vehicle/DSS treatment on day 12 (Table 6).

Water consumption values were generally similar in all of the treatment groups from days 7 to 12. However, on study day 13, water consumption in the vehicle/DSS treatment group (2.0 ± 0.2 ml/day) was significantly less (P < 0.05) than in all the other treatment groups. Water consumption ranged between 3.5 ± 3 and 4.5 ± 0.3 ml/day.

Treatment of mice with M-17 (alone, or in combination with metronidazole) resulted in less shortening in colon length, as compared to that found in the vehicle/DSS-treated mice. On day 13, the colon length values (cm) were: 7.1 ± 0.2 (vehicle/water), 6.4 ± 0.1 (vehicle/DSS), 7.2 ± 0.1 (EC-M-17/DSS), 6.8 ± 0.2 (metronidazole/DSS), and 7.3 ± 0.1 (EC-M-17 plus metronidazole/DSS). All of the colon length values in the probiotic and metronidazole treatment groups were significantly (*P* < 0.05) longer than the value found in the vehicle/DSS treatment group.

**Table 6: DAI Time Course Data - M-17 and/or Metronidazole**

| **Study Day** | **Saline/Water (n = 6)** | **Saline/DSS (n = 9)** | **M-17/DSS (n = 10)** | **Metro/DSS (n = 10)** | **M-17+Metro/DSS (n = 10)** |
|---|---|---|---|---|---|
| 0 | 0 ± 0 | 0 ± 0 | 0 ± 0 | 0 ± 0 | 0 ± 0 |
| 2 | 0 ± 0 | 0.1 ± 0.1 | 0.4 ± 0.2 | 0 ± 0 | 0 ± 0 |
| 4 | 0 ± 0 | 0.2 ± 0.2 | 0.1 ± 0.1 | 0.3 ± 0.2 | 0.1 ± 0.1 |
| 6 | 0.2 ± 0.2 | 0.3 ± 0.2 | 0.1 ± 0.1 | 0.2 ± 0.1 | 0 ± 0 |
| 8 | 0.3 ± 0.2 | 0.3 ± 0.2 | 0 ± 0 | 0.1 ± 0.1 | 0 ± 0 |
| 10 | 0.2 ± 0.2 | 0.6 ± 0.2 | 0.7 ± 0.3 | 0.7 ± 0.2 | 0.8 ± 0.2 |
| 12 | 0.2 ± 0.2 | 1.6 ± 0.2 * | 0.3 ± 0.2 † | 0.6 ± 0.2 † | 0.3 ± 0.2 † |
| 13 | 0 ± 0 | 1.9 ± 0.1 * | 0.6 ± 0.2 † | 1.0 ± 0.2 † | 0.4 ± 0.2 † |

### EXAMPLE 3

### Combined effects of M-17 and Metronidazole treatment on DSS-induced Colitis

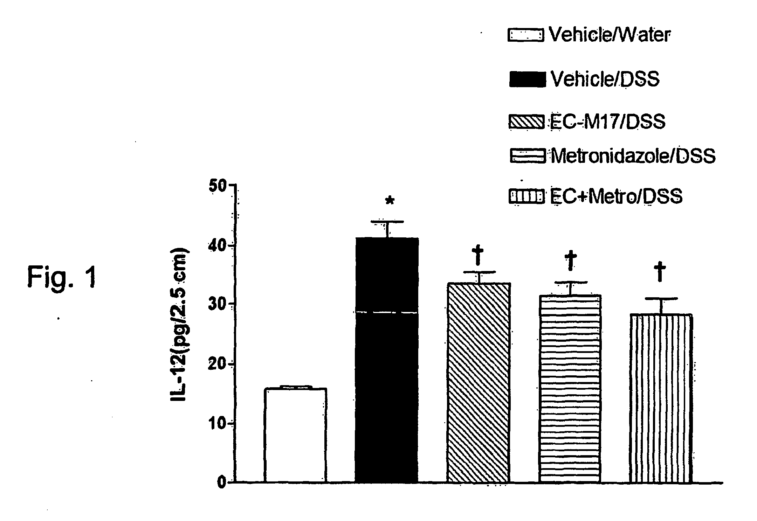

The effects of M-17 and Metronidazole, separately and in combination, on DSS-induced colitis were studied. The results are presented in Figures 1-6, and in Table 7 below. As shown in Table 7, either of these treatments alone reduced colonic levels of IL-12, IL-6, IL-1β, and IFN-γ, as well as changes in DAI, colon length, colon weight, MPO activity, and colonic histology score. No reductions in levels of IL-10 or IL-4 were seen. A greater decrease in parameters of colitis was observed with combined M-17 and metronidazole treatment, indicating a synergistic effect of this combined treatment.

As shown in Figure 1, the administration of 2 % DSS led to an increase of about 2.6 fold in the mean colonic IL-12 level. On study day 13, mice treated with M-17, metronidazole, or a combination thereof exhibited significantly lower levels of colonic IL-12 as compared to saline treated mice. In mice treated with both M-17 and metronidazole, there was a reduction of greater than 50 % in the colonic IL-12 level.

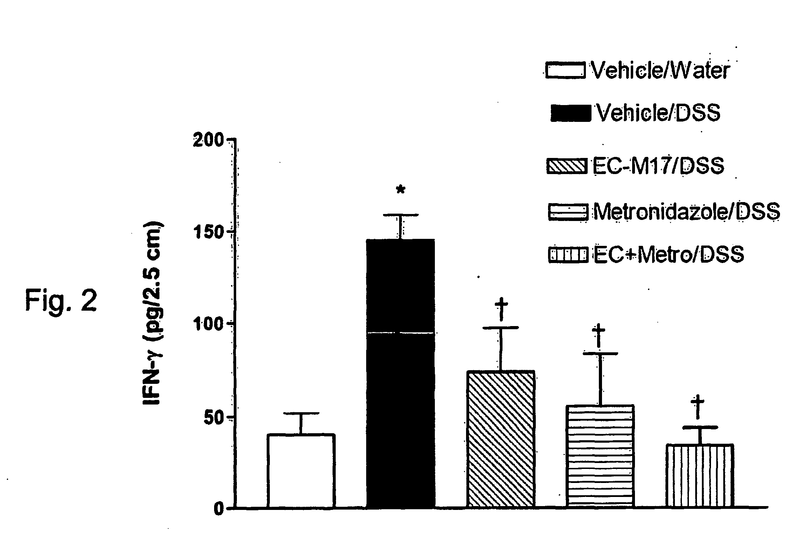

Similarly, following DSS treatment, an increase of about 3.5 fold in colonic IFN-γ was seen, as demonstrated in Figure 2. Animals treated with M-17, metronidazole, or combinations thereof had significantly lower levels of colonic IFN-γ (*P* < 0.05 vs. saline/DSS). In mice treated with both the probiotic and the antibiotic, IFN-γ levels were approximately the same as those seen in saline/water treated animals.

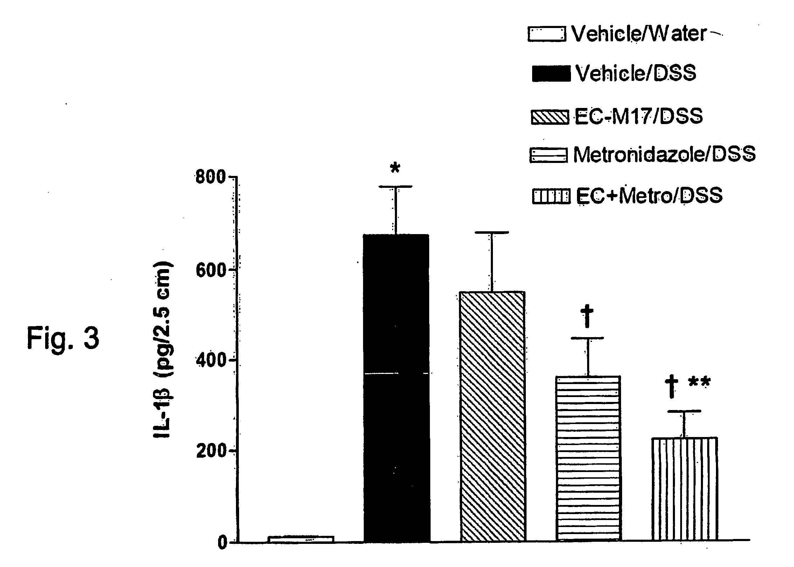

The ingestion of DSS resulted in an increase of greater than 50 % in the mean colonic IL-1β level, as shown in Figure 3. Mice treated with M-17, metronidazole, or a combination thereof, had lower levels of colonic IL-1β as compared to saline treated mice. In mice treated with both M-17 and metronidazole, a 67 % (P < 0.05) decrease in colonic IL-1β was seen.

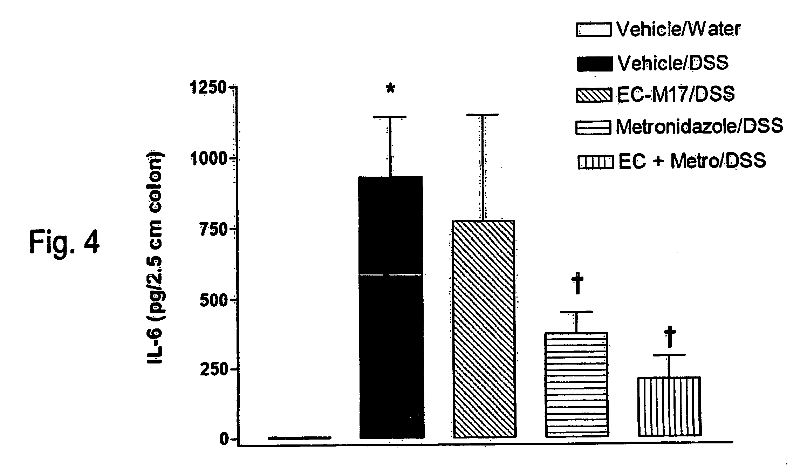

A very large (>200-fold) increase in colonic IL-6 was seen in DSS treated mice, as demonstrated in Figure 4. Animals treated with M-17 showed an overall reduction in colonic IL-6 content. However, a high IL-6 content was seen in the colon of a single probiotic treated mouse, which was responsible for the large standard error obtained in this study. Hence, statistical significance was not attained. Animals treated with metronidazole or both metronidazole and M-17 had significantly lower levels of colonic IL-6 as compared to saline treated mice. Animals treated with both M-17 and metronidazole showed a 78 % reduction in colonic IL-6 content.

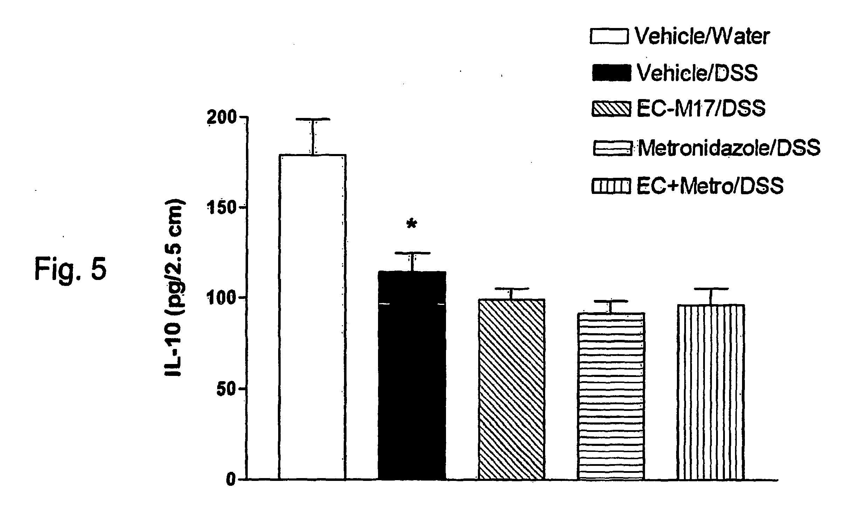

As shown in Figure 5, colonic IL-10 was attenuated in saline treated mice receiving DSS, as compared to saline treated mice. However, there was no evidence that probiotic and antibiotic treatment regimens increased the IL-10 level beyond that found in saline/DSS treated mice.

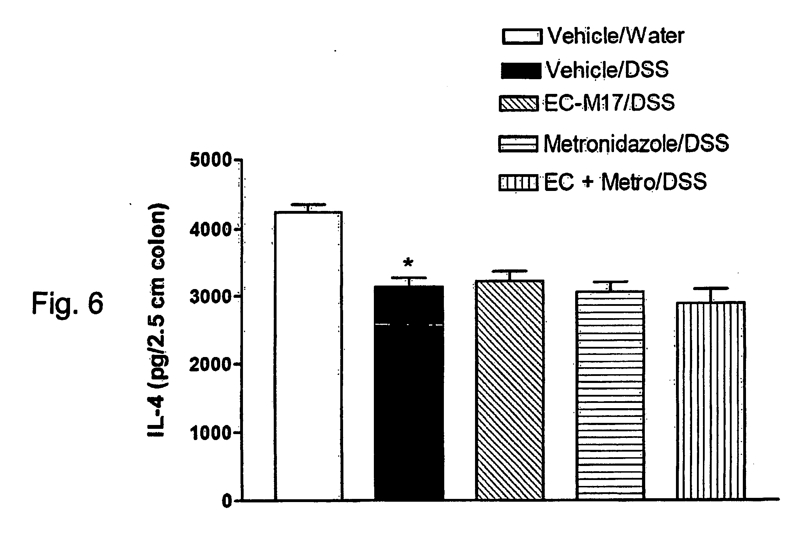

Similarly, as shown in Figure 6, colonic IL-4 was attenuated in saline/DSS treated mice, with no evidence that probiotic and antibiotic treatment regimens increased the IL-4 level beyond that found in saline/DSS treated mice.

**Table 7: Acute Phase DSS-Colitis Model (Day 13)**

| **Parameter** | **Saline/Water (n = 6)** | **Saline/DSS (n = 9)** | **M-17/DSS (n=10)** | **Metro/DSS (n =10)** | **M-17+Metro/DSS (n=10)** |
|---|---|---|---|---|---|
| **Modified DAI (0-4)** | 0 ± 0 | 1.9 ± 0.1 | 0.6 ± 0.2 * | 1.0 ± 0.2 * | 0.4 ± 0.2 * |
| **Colon length (cm)** | 7.1 ± 0.2 | 6.4 ± 0.1 | 7.2 ± 0.1 * | 6.8 ± 0.2 | 7.3 ± 0.1 * |
| **Colon wt. (mg/2.5 cm)** | 64 ± 3 | 84 ± 4 | 74 ± 3 * | 70 ± 3 | 66 ± 3 * |
| **MPO (U/2.5 cm)** | 1.0 ± 0.3 | 7.6 ± 0.9 | 5.5 ± 0.8 | 4.3 ± 1.0 * | 3.4 ± 0.5 * |
| **Histology Score (0-40)** | 4.5 ± 0.4 | 17.9 ± 1.3 | 11.2 ± 1.1 * | 12.4 ±0.9 * | 9.3 ± 0.7 * |
| **IFN-γ (pg/2.5 cm)** | 41 ± 11 | 145 ±14 | 74 ± 24 * | 56 ± 28 * | 35 ± 9 * |
| **IL-1β (pg/2.5 cm)** | 13 ± 1 | 672 ± 106 | 548 ± 129 | 359 ± 85 * | 224 ± 58 * |
| **IL-12 (pg/2.5 cm)** | 16 ± 0.4 | 41 ± 3 | 34 ± 2 * | 32 ± 2 * | 28 ± 3 * |
| **IL-6 (pg/2.5 cm)** | 4 ± 2 | 928 ± 213 | 773 ± 373 | 372 ± 75 * | 205 ± 83 * |
| **IL-10 (pg/2.5 cm)** | 179 ± 20 | 114 ± 10 | 99 ± 6 | 91 ± 7 | 96 ± 9 |

### EXAMPLE 4

### Effects of M-17 on Intestinal Permeability and Histology

Changes in intestinal permeability and their relationship to increases in DAI were investigated in DSS-treated mice receiving M-17. As shown in Figure 7, mice receiving saline and DSS showed a significant increase in colonic MPO activity. These data indicate increased colonic neutrophil influx in these animals (*Fitzpatrick et al.*, *2000*; *Fitzpatrick et al.*, *2002*). Mice treated with M-17, metronidazole or a combination thereof showed reduced levels of MPO. The combined treatment regimen reduced colonic MPO by about 55 (P < 0.05) %. Interestingly, the combined treatment regimen also significantly reduced colonic MPO by 38.2% (P < 0.05) compared with M-17 treatment alone.As shown in Figure 8, increases in DAIs were observed in DSS-treated mice, which were reduced by treatment with M-17. No increase in DAIs was demonstrated with M-17 alone, in the absence of DSS.

Figure 9 shows a correlation between the increases in intestinal permeability and in DAIs in saline-treated mice following DSS-induction of colitis, with no M-17 treatment.

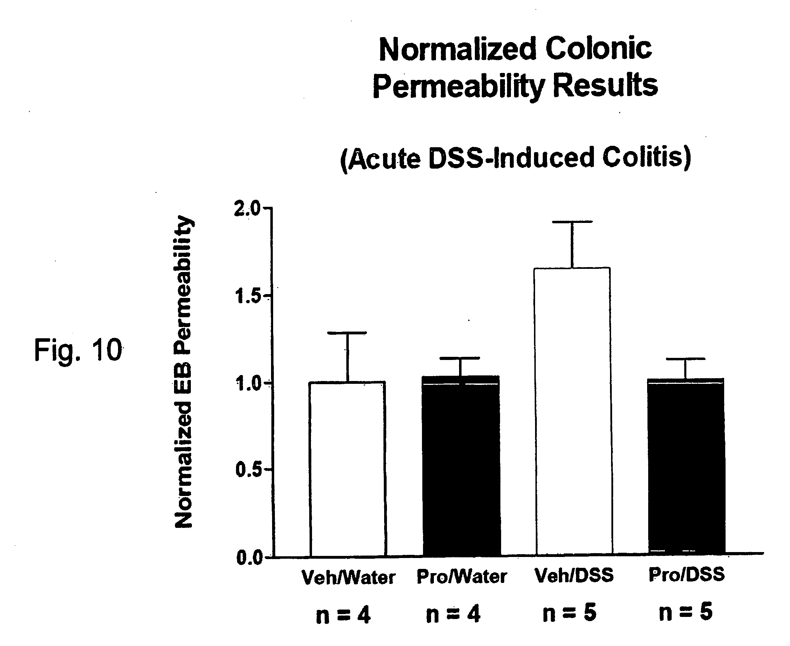

In contrast to Figure 7, Figure 10 shows that normalized intestinal permeability values were substantially identical in mice treated with M-17/DSS and M-17/Water. Therefore, there was no significant correlation between intestinal permeability and DAI parameters in M-17-treated mice.

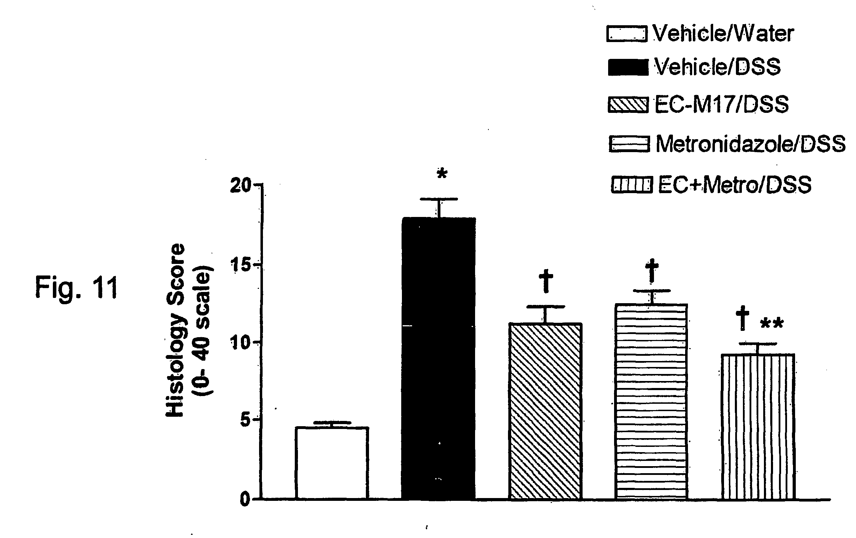

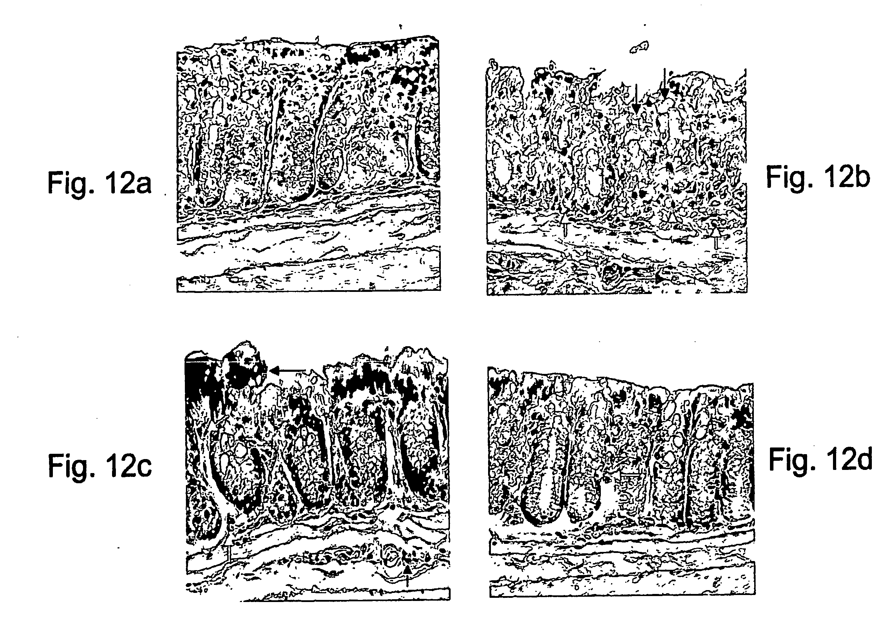

As shown in Figures 11 and 12, saline/DSS treated mice showed greater histological damage than those receiving water throughout the study. Treatment of mice with the probiotic, antibiotic, or a combination thereof, resulted in significant decreases in the total colonic histology scores (Figure 11).

Representative photographs from this study are shown in Figures 12A-12D. Figure 12A shows histological results obtained from a control animal treated with saline/water. As shown in Figure 12B, administration of DSS resulted in crypt damage (indicated by black arrows), significant numbers of inflammatory cells in the lamina propria (white arrows), as well as inflammatory cells in the submucosa (gray arrow). In this representative saline/DSS treated mouse, inflammatory cells were also seen at the luminal surface. As shown in Figure 12C, in a representative M-17 treated mouse, evidence of some surface epithelial cell damage (black arrow), as well as mild inflammatory cell influx in the lamina propria (white arrows) and submucosa (gray arrows) was also seen. In an animal treated with M-17 in combination with metronidazole (Figure 12D), the crypt architecture was preserved, with only mild inflammatory cell infiltration in the lamina propria (white arrow). The pattern of colonic histology in this animal generally resembled that found in the saline/water treated mouse of Figure 12A.

### EXAMPLE 5

### Effects of M-17 on LMP2 Expression

Up-regulation of LMP2 was observed in DSS-treated mice, as compared to the water-treated group, as indicated by Western blot studies of colonic LMP2 expression, and further shown in Figures 13 and 14. Treatment with M-17 did not affect colonic LMP2 expression, except in one individual mouse, which cannot be considered representative of the group as a whole.

### EXAMPLE 6

### Sensitivity of E. coli ATCC 202226 to metronidazole

The Kirby-Bauer disc diffusion method was used to determine the relative sensitivity of *E*. *coli* ATCC 202226 to metronidazole (*Bauer*, *1966*). Five colonies of *E. coli* ATCC 202226 were picked from trypticase soy agar (TSA) using a sterile inoculation loop and suspended in trypticase soy broth (TSB). The TSB culture was grown for 6 hours at 37° C/200 rpm. This culture was diluted in TSB to match a 0.5 McFarland turbidity standard prior to saturating a sterile cotton swab and streaking the culture onto the surface of a Mueller-Hinton Agar plate. After allowing the surface of the plate to dry, a 50 µg metronidazole paper disc (Oxoid) was placed on the lawn. The plate was incubated overnight and the zone of inhibition was recorded.

No zone of inhibition was observed surrounding the metronidazole disc, indicating that E. *coli* ATCC 202226 has a high level of resistance against this antibiotic.

### EXAMPLE 7

### Insights on the mechanism of action of a biotherapeutic composition of M-17 and metronidazole

It has previously been suggested that there are five major mechanisms of action for probiotics in therapy for IBD: immunomodulatory actions; enhanced intestinal barrier integrity; antimicrobial activity; stimulation of an immune response; and competitive exclusion of bacterial adhesion/translocation (*Fedorak*, *2004*). In this regard, M-17 appears to have immunomodulatory actions, because it can decrease levels of pro-inflammatory cytokines, which are increased in conjunction with murine colitis. This probiotic agent did not consistently modulate the expression of colonic LMP2, which is a proteasome subunit that is thought to play roles in antigen presentation and NF-κB signaling (Figure 14).

The data presented in Figures 7 and 10 do not support a direct role for M-17 in enhancing intestinal barrier integrity. In fact, intestinal permeability was enhanced in probiotic/water treated mice. However, the normalized intestinal permeability value was virtually identical (i.e., did not change) in the M-17/DSS group, as compared to the M-17/Water group (Figure 10). These results suggest that the probiotic may prevent a secondary increase in permeability associated with DSS administration. In this regard, pro-inflammatory cytokines are known to disrupt intestinal barrier permeability (*Ma*, *2004*; *Sappington*, *2003*). M-17 may prevent a secondary increase in permeability, by attenuating the production of inflammatory cytokines that occur during DSS administration (see Table 7).

The overall conclusion from these studies is that an oral dose of 5 x 10⁹ CFU/ml and 5 ml/kg, *E. coli* Strain M-17 caused no major deleterious side effects in C57/BL-6 mice. At an oral dose of 5 x 10⁹ CFU/ml, M-17 attenuated various parameters of DSS-induced colitis in C57/B1L-6 mice. These parameters included a clinical marker (Disease Activity Index), morphological markers (colon length, segmental colon weight), and biochemical markers (colonic myeloperoxidase and pro-inflammatory cytokine levels). M-17 appeared to have a similar efficacy profile to metronidazole for reducing parameters of acute DSS-induced colitis. Combined administration of EC-M-17 and metronidazole was highly effective in reducing parameters of colitis in C57/BL-6 mice.

### EXAMPLE 8

### Effects of M-17 on fecal microbiology

Fecal samples were found to contain mainly gram positive bacteria belonging to the general category of lactic acid bacteria (*Lactobacillus*, *Leuconostoc*, *Enterococcus*, *Staphylococcus and Streptococcus*). The gram positive bacteria outnumbered the gram negative bacteria by about 100-fold. The number of gram negative bacteria in all samples ranged from about 2x10⁶ to about 1 x10⁷ CFUs per gram wet weight of the fecal pellet, with the number of gram-negative bacteria in the fecal pellets from the untreated (Saline/Water) mice being slightly, but not significantly, less that of all other groups. The prevailing gram negative flora of the untreated mice consisted of the aerobic *Pseudomonas,* with occasional *Escherichia coli* and *Enterobacter*.

Mice to which DSS was administered displayed an increase in the proportion *Enterobacter* gram negative bacteria and a decrease in the proportion of *Escherichia coli.* Paradoxically, the fecal samples from mice treated with M-17 probiotic and DSS displayed virtually no *E*. *coli* colonies. The flora of mice treated with metronidazole alone and DSS was essentially the same as that of the samples from mice treated with saline and DSS. The aerobic flora of mice treated with metronidazole, M-17 and DSS differed from that of animals not treated with M-17, in that an unidentified facultative gram negative bacterium was found in three out of nine (33%) of the fecal samples.

Metronidazole, when administered *ex vivo* at 50 µg/ml, did not inhibit individual isolates of the aerobic and facultative gram positive and gram negative flora.

### EXAMPLE 9

### Effects of M-17 on colonic NF-κB p65

Expression of the NF-κB p65 subunit in the DSS colitis model was studied. Representative western blots from mouse colonic nuclear extracts are shown in Figure 15. As can be seen, relatively little expression of p65 was found in the colons of animals receiving water over a 13-day period. In contrast, more prominent p65 expression was found in animals receiving 2 % saline/DSS. However, the expression of p65 was less evident in mice from the probiotic M-17/DSS treatment group. A relative densitometry analysis, whereby the data were normalized to the mean p65 value found in saline/Water treated mice, yielded evidence that p65 expression was enhanced in saline/DSS treated animals, and that treatment of mice with M-17 for 13 days reduced the mean expression of p65.

### IN VITRO STUDIES

### EXAMPLE 10

### Effects of M-17 on the NF-κB reporter gene assay

An initial dose response study showed that M-17 attenuated TNF-α induced activation of a NF-κB driven luciferase reporter gene assay system in a dose dependant manner (Figure 16A). At 5x10⁷ CFU/ml, some inhibition (29%, *P* < 0.05) of NF-κB signaling was evident. More profound inhibition (89%, *P* < 0.001) was seen with a 5 x 10⁸ CFU/ml dose of M-17, as well as with a 5 x 10⁹ CFU/ml dose of M-17 (96% inhibition, *P* < 0.001). 5 x 10⁹ CFU/ml is also the concentration of M-17 utilized in the *in vivo* colitis model. In a follow-up study (Figure 16B), 1 x 10⁸ CFU/ml of M-17 were administered. Even at this relatively low concentration, which was 50-fold less than the concentration used in the *in vivo* study with DSS-induced colitis, TNF-α induced activation of NF-κB was significantly reduced (65%, *P* < 0.05) by the probiotic. At this concentration, M-17 had no effect on the viability of the human embryonic kidney 293T cells, as determined by the MTS mitochondrial metabolism assay (Promega).

### EXAMPLE 11

### Effects of E. coli strains on macrophage activation

The effects of probiotic *E. coli* strains on macrophage activation were studied *in vitro* using the mouse macrophage cell line RAW 264.7, as shown in schematic form in Figure 17.

RAW 264.7 were obtained from American Type Culture Collection (ATCC), and grown in Dulbecco's Modified Eagle's Medium (DMEM) containing 10% FBS (Fetal Bovine Serum).

Naïve macrophages were utilized at a density of 2 million per ml. *E*. *coli* strains ECOR-29, ECOR-49; ECOR-51, ECOR-59, ECOR-61, ECOR-62 or M-17 were added to the macrophage cell culture system, at a concentration of about 1x10⁸ CFU/ml. Immediately afterwards, lipopolysaccharide (LPS) was added at 5 µg/ml. Control groups were exposed to saline, in the presence and absence of the above-listed *E*. *coli* strains, and to the *E. coli* strain alone.

Samples of culture medium were collected immediately, and after 4 hours, and levels of cytokines, IL-1β, 1L-6 and TNF were assayed, using the appropriate ELISA kits (Pierce-Endogen), according to the directions in the kit.

As shown in Figure 18, IL-1β secretion by the macrophage cell line RAW 264.7 in the presence of either saline alone or LPS was increased significantly in the presence of *E*. *coli* strain ECOR-51, while other strains had only a minor effect on secretion levels. LPS treatment resulted in a significant increase in IL-1β secretion, which was attenuated by the presence of all *E*. *coli* strains studied, with the exception of ECOR-51. The greatest level of attenuation was shown with strains ECOR-59 and M-17.

As shown in Figure 19, TNF-α secretion in the presence of saline was greatly increased in the presence of all *E*. *coli* strains studied, except for M-17. LPS alone resulted in a very large increase in TNF level, which was attenuated to some extent by all strains studied, with the greatest attenuation occurring in the presence of M-17.

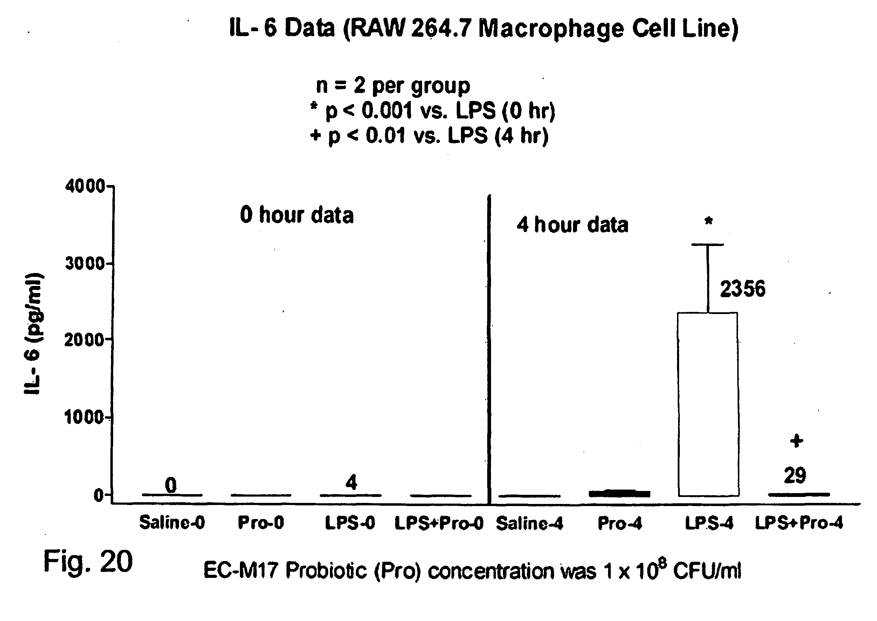

As shown in Figure 20, levels of IL-6 were greatly increased in culture samples obtained 4 hours after stimulation with LPS, while control samples treated with saline or with M-17 alone showed no significant increase. The LPS-stimulated increase was almost totally blocked by addition of M-17.

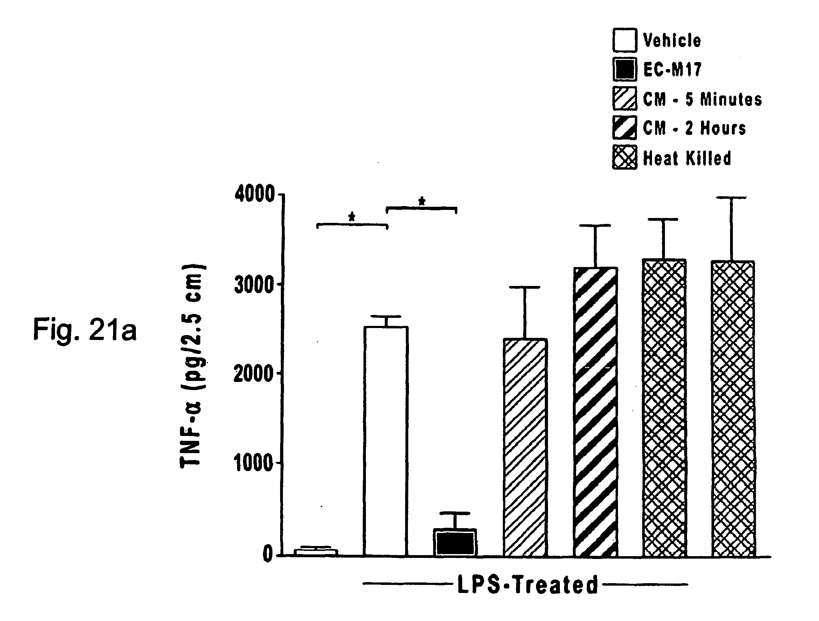

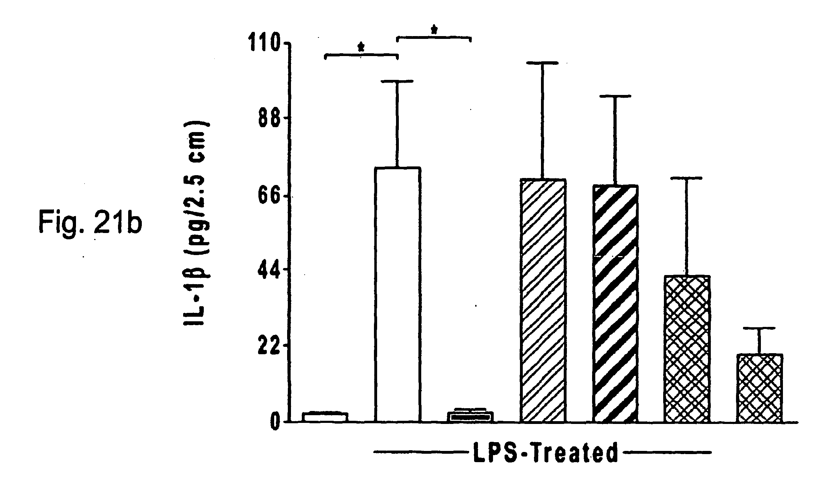

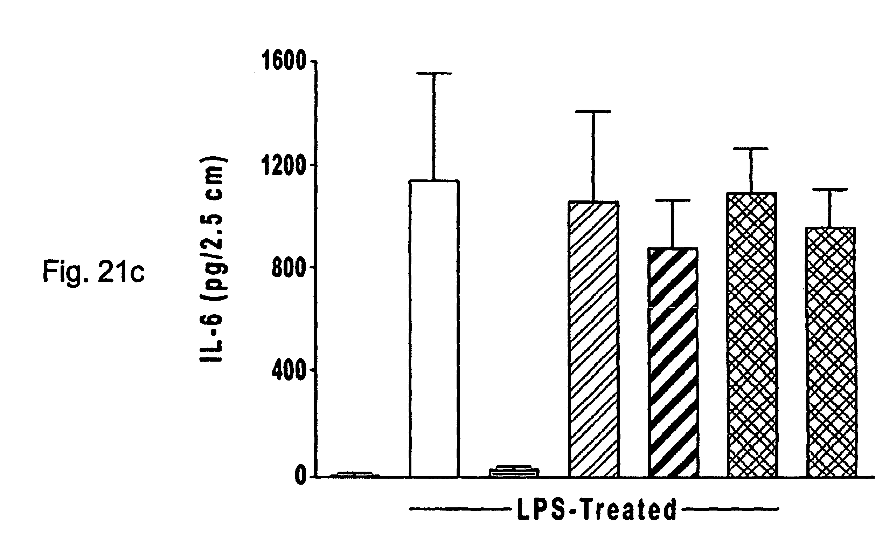

As shown in Figures 18-20, cytokine secretion in this macrophage cell line was essentially near zero at baseline. After 4 hours, M-17 only mildly stimulated secretion of the three cytokines assayed, whereas treatment with LPS resulted in substantial increases in the secretion of pro-inflammatory cytokines (Figures 18-20). M-17 significantly inhibited (> 90 %, *P* < 0.05) the LPS-induced secretion of TNF-α, IL-1β and lL-6 when compared with LPS-treated cells exposed only to vehicle. The concentrations of these cytokines were virtually the same as those observed with M-17 treatment alone. By comparison, metronidazole (50 µg/ml), when exposed to RAW 264.7 cell cultures, did not reduce LPS-stimulated cytokine secretion (data not shown). Neither heat-killed M-17 nor M-17 conditioned media effectively reduced cytokine secretion in LPS-treated macrophages (Figure 21). At 1 x 10⁸ CFU/ml, M-17 did not affect macrophage viability as determined by the trypan blue exclusion method.

### EXAMPLE 12

### Effects of M-17 on p65 nuclear binding in macrophages

After 3 hours of exposure, M-17 alone (without LPS) only mildly increased the nuclear binding of p65 compared with vehicle, However, the Lops-induced increase in p65 binding was significantly inhibited (78%, *P* < 0.05) with 1 x 10⁸ CFU/ml of M-17 (Figure 22). A confirmation of assay specificity was demonstrated by complete inhibition of p65 binding by a wild type-oligonucleotide control.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### BIBLIOGRAPHY

Araki, Y., et al. Gastroenterology 35: 1060-1067 (2000).
Araki, Y., et al. Int. J. Mol. Med 13: 577-580 (2004).
Baldwin, A.S. Annu. Rev. Immunol. 14: 649-683 (1996).
Bauer, A.W., et al. Am. J. Clin. Pathol. 45(4): 493-496 (1966).
Bauerle, P.A. and Henkel, T. Annu. Rev. Immunol. 12: 141-179 (1994).
Caradonna, L., et al. J. Endotoxin. Res. 6: 205-214 (2000).
Chen, C.N., et al. Am. J. Surg. 183: 464-470 (2002).
Egger, B., et al. Digestion 62: 240-248 (2000).
Fitzpatrick, L.R., et al. Dig. Dis. Sci. 45: 2327-2336 (2000).
Fitzpatrick, L.R. et al. Inflamm. Bowel Dis. 8: 159-167 (2002)
Fitzpatrick, L.R., et al. Gastroenterology 126: A417 (2004).
Gaudio, E., et al. Dig. Dis. Sci. 44: 1458-1475 (1999).
Jobin, C. and Sartor, R.B. Inflam. Bowel Dis. 6: 206-213 (2000a).
Jobin, C. and Sartor, R.B. Am. J. Cell Physiol. 278: C451-C462 (2000b).
Kitajima, S., et al. Exp. Anim. 48: 137-143 (1999).
Krieglstein, C.F., et al. J. Surg. Res. 101: 166-175 (2001).
Kruis, W., et al. Gut 53: 1617-1623 (2004).
Lee, J.I. and Burckart, G.J. J. Clin. Pharmacol. 38: 981-993 (1998).
Levin, K.E., et al. Dis. Colon Rectum 35: 452-456 (1992).
Madden, M.V., et al. Dig. Dis. Sci. 39: 1193-1196 (1994).
Monteleone, G., et al. J. Clin. Investigation 108: 601-609 (2001).
Murano, M., et al. Clin. Exp. Immunol. 120: 51-58 (2000).
Murthy, S.N., et al. Dig. Dis. Sci. 38: 1722-1734 (1993).
Nasmyth, D.G., et al. Gastroenterology 96: 817-824 (1989).
Okayasu, I., et al. Gastroenterology 98: 694-702 (1990).
Osman, N., et al. Dig. Dis. Sci. 49: 320-327 (2004).
Patel, R.T., et al. Dis. Colon Rectum 38: 831-837 (1995).
Shen, B., et al. Inflamm. Bowel Dis. 7: 301-305 (2001).
Schottelius, A.J.G. and Baldwin, A.S. Int. J. Colorectal Dis. 14: 18-28 (1999).
Schouten, W.R. Mediators of Inflammation 7: 175-181 (1998).
Spiik, A. K., et al. Int. J. Colorectal Dis 17: 223-232 (2002).
Strober, W., et al. Annu. Rev. Immunol. 20: 495-549 (2002).
Tak, P.P. and Firestein, G.S. J. Clin. Invest. 107: 7-11 (2001).
Williams, K.L, et al. Gastroenterology 120: 925-937 (2001).
Wolf and Lashner, Cleveland Clinic Journal of Medicine 69: 621-631 (2002).

## Claims

1. A biotherapeutic composition comprising a pharmaceutically effective amount of a probiotic *Escherichia coli* strain, a pharmaceutically effective amount of an anaerobic bacteria antibiotic to which said *Escherichia coli* strain is resistant, and a pharmaceutically acceptable carrier, wherein said antibiotic is metronidazole, and wherein said *Escherichia coli* strain and said antibiotic act in synergy.

2. The composition of claim 1, wherein said *Escherichia coli* strain is selected from the group consisting of M-17, an isolate thereof and a mutant thereof.

3. The composition of claim 2, wherein said isolate or mutant of *Escherichia coli* strain M-17 is selected from the group consisting of *Escherichia coli* strain BU-230-98 ATCC Deposit No. 202226 (DSM 12799), a nalidixic acid resistant strain of M-17 and *Escherichia coli* strain ATCC Deposit No. PTA-7295(M-17_{SNAR}).

4. The composition of claim 1, packaged in a packaging material and identified in print, in or on said packaging material, for use in the treatment or prevention of a condition caused by an anaerobic bacterium, wherein said anaerobic bacterium is a sulfate-reducing bacterium and wherein said condition is an intestinal disorder.

5. Use of a pharmaceutically effective amount of a probiotic *Escherichia coli* strain, in combination with a pharmaceutically effective amount of an anaerobic bacteria antibiotic to which said *Escherichia coli* strain is resistant, said antibiotic being metronidazole, in the manufacture of a medicament for treating a condition caused by an anaerobic bacterium, wherein said *Escherichia coli* strain and said antibiotic act in synergy and wherein said anaerobic bacterium is a sulfate-reducing bacterium and wherein said condition is an intestinal disorder.

6. The use of claim 5, wherein said pharmaceutically effective amount of said metronidazole is about 20 mg per kg body weight of said subject.

7. The use of claim 5, wherein said antibiotic and said probiotic *Escherichia coli* strain together form a part of a biotherapeutic composition which further comprises a pharmaceutically acceptable carrier.

8. The composition of claim 4 or the use of claim 5, wherein said condition is pouchitis.

9. The use of claim 5, wherein said condition is a combination of ulcerative colitis and pouchitis.

## Patentansprüche

1. Biotherapeutische Zusammensetzung, umfassend eine pharmazeutisch wirksame Menge eines probiotischen *Escherichia coli*-Stamms, eine pharmazeutisch wirksame Menge eines anaeroben Bakterien-Antibiotikums, gegen das der *Escherichia coli*-Stamm resistent ist, und einen pharmazeutisch verträglichen Träger, wobei das Antibiotikum Metronidazol ist und wobei der *Escherichia coli*-Stamm und das Antibiotikum synergistisch agieren.

2. Zusammensetzung nach Anspruch 1, wobei der *Escherichia coli*-Stamm ausgewählt ist aus der Gruppe bestehend aus M-17, einem Isolat davon und einer Mutante davon.

3. Zusammensetzung nach Anspruch 2, wobei das Isolat oder die Mutante des *Escherichia coli*-Stamms M-17 ausgewählt ist aus der Gruppe bestehend aus dem *Escherichia coli*-Stamm BU-230-98 mit der ATCC-Hinterlegungsnummer 202226 (DSM 12799), einem Nalidixinsäure-resistenten Stamm von M-17 und dem *Escherichia coli*-Stamm mit der ATCC-Hinterlegungsnummer PTA-7295 (M-17_{SNAR}).

4. Zusammensetzung nach Anspruch 1, die in Verpackungsmaterial verpackt ist und die in oder auf dem Verpackungsmaterial gedruckt für die Verwendung in der Behandlung oder Vorbeugung einer Erkrankung identifiziert ist, die von einem anaeroben Bakterium verursacht wird, wobei das anaerobe Bakterium ein Sulfat-reduzierendes Bakterium ist und wobei die Erkrankung eine Darmerkrankung ist.

5. Verwendung einer pharmazeutisch wirksamen Menge eines probiotischen *Escherichia coli*-Stamms, in Kombination mit einer pharmazeutisch wirksamen Menge eines anaeroben Bakterien-Antibiotikums, gegen das der *Escherichia coli*-Stamm resistent ist, wobei das Antibiotikum Metronidazol ist, für die Herstellung eines Medikamentes zur Behandlung einer Erkrankung, die von einem anaeroben Bakterium verursacht wird, wobei der *Escherichia coli*-Stamm und das Antibiotikum synergistisch agieren, wobei das anaerobe Bakterium ein Sulfat-reduzierendes Bakterium ist und wobei die Erkrankung eine Darmerkrankung ist.

6. Verwendung nach Anspruch 5, wobei die pharmazeutisch wirksame Menge an Metronidazol etwa 20 mg pro kg Körpergewicht des Probanden ist.

7. Verwendung nach Anspruch 5, wobei das Antibiotikum und der probiotische *Escherichia coli*-Stamm zusammen einen Teil einer biotherapeutischen Zusammensetzung bilden, die zusätzlich einen pharmazeutisch verträglichen Träger umfasst.

8. Zusammensetzung nach Anspruch 4 oder Verwendung nach Anspruch 5, wobei die Erkrankung Pouchitis ist.

9. Verwendung nach Anspruch 5, wobei die Erkrankung eine Kombination aus Colitis ulcerosa und Pouchitis ist.

## Revendications

1. Composition biothérapeutique comprenant une quantité pharmaceutiquement efficace d'une souche *Escherichia coli* probiotique, une quantité pharmaceutiquement efficace d'un antibiotique de bactéries anaérobies auquel ladite souche *Escherichia coli* est résistante, et un support pharmaceutiquement acceptable, dans laquelle ledit antibiotique est le métronidazole, et dans laquelle ladite souche *Escherichia coli* et ledit antibiotique agissent en synergie.

2. Composition selon la revendication 1, dans laquelle ladite souche *Escherichia coli* est choisie dans le groupe constitué de M-17, un isolat de celle-ci et un mutant de celle-ci.

3. Composition selon la revendication 2, dans laquelle ledit isolat ou mutant de la souche *Escherichia coli* M-17 est choisi dans le groupe constitué de la souche *Escherichia coli* BU-230-98 numéro de dépôt ATCC 202226 (DSM 12799), une souche de MP-17 résistante à l'acide nalidixique et la souche *Escherichia coli* numéro de dépôt ATCC PTA-7295 (M-17_{SNAR)}.

4. Composition selon la revendication 1, empaquetée dans un matériau d'empaquetage et identifiée par marquage, dans ou sur le matériau d'empaquetage, pour une utilisation dans le traitement ou la prévention d'une maladie causée par une bactérie anaérobie, dans laquelle ladite bactérie anaérobie est une bactérie réduisant le sulfate et où ladite maladie est un trouble intestinal.

5. Utilisation d'une quantité pharmaceutiquement efficace d'une souche *Escherichia coli* probiotique, en combinaison avec une quantité pharmaceutiquement efficace d'un antibiotique de bactéries anaérobies auquel ladite souche *Escherichia coli* est résistante, ledit antibiotique étant le métronidazole, dans la fabrication d'un médicament pour le traitement d'une maladie causée par une bactérie anaérobie, où ladite souche *Escherichia coli* et ledit antibiotique agissent en synergie et où ladite bactérie anaérobie est une bactérie réduisant le sulfate et où ladite maladie est un trouble intestinal.

6. Utilisation selon la revendication 5, où ladite quantité pharmaceutiquement efficace dudit métronidazole est d'environ 20 mg par kg en poids dudit sujet.

7. Utilisation selon la revendication 5, où ledit antibiotique et ladite souche *Escherichia coli* probiotique forment ensemble une partie d'une composition biothérapeutique qui comprend en outre un support pharmaceutiquement acceptable.

8. Composition selon la revendication 4 ou utilisation selon la revendication 5, où ladite maladie est la pouchite.

9. Utilisation selon la revendication 5, où ladite maladie est une combinaison de rectocolite hémorragique et de pouchite.
